Europäisches Patentamt

(19) European Patent Office

Office européen des brevets

(11) Publication number: **0 123 485**
A1

## (12) EUROPEAN PATENT APPLICATION

(21) Application number: **84302549.5**

(22) Date of filing: **13.04.84**

(51) Int. Cl.³: **A 61 K 31/70**
A 61 K 31/705, C 07 J 17/00

(30) Priority: **14.04.83 US 484983**
**29.03.84 US 593492**

(43) Date of publication of application:
**31.10.84 Bulletin 84/44**

(84) Designated Contracting States:
**AT BE CH DE FR GB IT LI LU NL SE**

(71) Applicant: **THE REGENTS OF THE UNIVERSITY OF CALIFORNIA**
**2199 Addison Street**
**Berkeley California 94720(US)**

(72) Inventor: **Friend, David Robert**
**65-28th Avenue No. 1**
**San Mateo, California 94403(US)**

(72) Inventor: **Chang, George Washington**
**1619 Josephine Street**
**Berkeley California 94703(US)**

(74) Representative: **Bizley, Richard Edward et al,**
**BOULT, WADE & TENNANT 27 Furnival Street**
**London EC4A 1PQ(GB)**

(54) Colon-specific prodrug, the preparation and uses thereof.

(57) A colon-specific drug delivery system is based on the use of a synthetic drug glycoside prodrug composition which, when ingested by a mammal, undergoes reaction with glycosidases produced by colonic microflora to release a free drug capable of being absorbed to or absorbed by the intestinal mucosa. Hitherto, synthetic drug glycosides useful in the delivery of chemotherapeutic agents to the colon were not known.

EP 0 123 485 A1

Croydon Printing Company Ltd.

COLON-SPECIFIC PRODRUG, THE PREPARATION AND USES THEREOF

This invention relates to a colon-specific drug delivery system. More specifically, the invention relates to a colon-specific drug delivery system based on the use of a synthetic drug glycoside prodrug composition which, when ingested by a mammal, undergoes reaction with glycosidases produced by colonic microflora to release a free drug capable of being adsorbed to or absorbed by the intestinal mucosa.

In recent years there has been increased emphasis on ways to deliver or activate drugs at specific sites in order to reduce side effects and increase pharmacological response. Implantable pumps, adhesive patches impregnated with drugs, vesicle enclosed drugs, and drug carriers have been proposed to achieve site-specific delivery. Another approach has been to use prodrugs (See Stella, V.J. and Himmelstein, K.J., J. Med. Chem. 23:1275-1282 (1980); Sinkula, A.A. and Yalkosky, S.H., J. Pharm. Sci. 64:181-210 (1975)) which, by virtue of their physicochemical properties, can reach specific sites and then be converted to the active drug in situ. The site-specific delivery of prodrugs administered systemically to the kidney, brain, breasts, or the central nervous system or topically to the eyes or skin have been reported. In all these cases, the parent drug is released chemically or by specific enzyme(s) present at the target site.

It is known that colon-specific delivery of bioactive compounds occurs in man. Anthraquinone cathartics and the carcinogen cyasin are naturally occurring glycosides found in plants. See Scheline, R.R., J. Pharm. Sci. 57:2021-2137 (1968). Upon ingestion, these substances pass unabsorbed to the large intestine, where the bioactive moieties are released by the enzymatic action of the colonic microflora. The azo-reductase activity of the colonic microflora is now known to activate certain sulfa drugs by reducing the azo-bond present in such compounds. See Mandel, G.L. and Sande, M.A. in "The Pharmacological Basis of Therapeutics", Sixth Ed. (A.G. Gilman, L.S. Goodman, and A. Gilman, Eds.) MacMillan, New York, N.Y., (1980) pp. 1106-1165. Also, the reduction of the azo-link between an unabsorbed polymer and certain aromatic amines form the basis of a recently developed colon-specific drug delivery system. See Parkinson, T.M., Brown, J.P., and Winegard, R.E., U.S. Patent 4,190,716 (Feb. 26, 1980); and Brown, J.P., Appl. Enviro. Microbiol. 41:1283-1286 (1981).

It is also known that the intestinal microflora have unique enzymatic activities capable of hydrolyzing a number of specific chemical bonds. See Drasar, B.S. and Hill, M.J., "Human Intestinal Flora", Academic Press, London, (1974) pp 54-71. However, prior to the present invention it was not known that following ingestion by a mammal, a colon-specific drug glycoside would pass through the stomach and small intestine without being significantly hydrolyzed by endogenous mucosal enzymes produced by the mammalian host. In addition, prior to the present invention it was not known that such a drug glycoside prodrug would pass into the area of the colon where it would be essentially cleaved by the glycosidase activity of colonic microflora. Prior to the teaching of the present invention, it was also not known that once such cleavage of the prodrug occurred, a free drug capable of being adsorbed to or absorbed by the intestinal mucosa would be released.

Such a synthetic prodrug comprised of a drug glycoside has advantages over the compounds used in the colon-specific delivery systems of the prior art. The fact that the prodrug is synthetic makes it possible to create drug glycosides not found in nature. For example, synthetic drug glycosides are disclosed herein that are useful in the treatment of ulcerative colitis, a condition which carries an increased risk of colon cancer, and other forms of inflammatory bowel disease. Natural drug glycosides useful for the treatment of inflammatory bowel disease are not found in nature. Synthetic drug glycosides of the type disclosed herein will also be useful in the delivery of chemotherapeutic agents to the colon and then to liver metastases with minimal damage to the stomach and small intestine. Synthetic drug glycosides useful in the delivery of chemotherapeutic agents to the colon are not found in nature.

The drug glycosides of the present invention utilize simple sugars as the disabling moiety that substantially prevents liberation or absorption of the free drug until the prodrug reaches the area of the colon. Colonic microfloral glycosidases then act upon the drug glycoside, liberating the sugar moiety from the drug moiety. These liberated sugar residues can be utilized as an energy source by the intestinal bacteria. Although the nonabsorbable polymeric backbone utilized in the colon-specific drug delivery system disclosed in U.S. Patent 4,190,716 may prove harmless to patients exposed to the polymers for considerable lengths of time, the long term effect of these compounds has not yet been evaluated. On the other hand, the colon-specific drug delivery system disclosed herein leaves no such unnatural waste compound for the body to contend with.

It is an aspect of the invention to provide a colon-specific drug delivery system useful for the treatment of disease that will utilize a synthetic prodrug comprised of a drug glycoside capable of being essentially cleaved by glycosidase enzymatic activity of colonic microflora but not capable of being significantly hydrolyzed by endogenous enzymes produced by the mammalian host, thus enabling the most significant amounts of free drug to be released in the area of the colon following cleavage of the drug glycoside by glycosidases produced by the colonic microflora.

It is a further aspect of the invention to provide a colon-specific drug delivery system useful for the treatment of disease that will utilize a synthetic prodrug comprised of a steroid glycoside capable of being essentially cleaved by glycosidase enzymatic activity of colonic microflora but not capable of being significantly hydrolyzed by endogenous enzymes produced by the mammalian host, thus enabling the most significant amounts of free drug to be released in the area of the colon following cleavage of the drug glycoside by glycosidases produced by the colonic microflora.

It is a further aspect of the invention to provide a colon-specific synthetic prodrug composition comprised of a steroid glycoside capable of being essentially cleaved by beta-galactosidase enzymatic activity of colonic microflora but not capable of being significantly hydrolyzed by endogenous enzymes produced by the mammalian host, thus enabling the most significant amounts of free drug to be released in the area of the colon following cleavage of the drug

-5-

glycoside by glycosidases produced by the colonic microflora.

It is a further aspect of the invention to provide a colon-specific synthetic prodrug composition comprised of a steroid glycoside capable of being essentially cleaved by alpha-galactosidase enzymatic activity of colonic microflora but not capable of being significantly hydrolyzed by endogenous enzymes produced by the mammalian host thus enabling the most significant amounts of free drug to be released in the area of the colon following cleavage of the drug glycoside by glycosidases produced by the colonic microflora.

It is a further aspect of the invention to provide a method for producing a colon-specific synthetic prodrug composition comprised of a steroid glycoside capable of being essentially cleaved by beta-glucosidase enzymatic activity of colonic microflora but not capable of being significantly hydrolyzed by endogenous enzymes produced by the mammalian host thus enabling the most significant amounts of free drug to be released in the area of the colon following cleavage of the drug glycoside by glycosidases produced by the colonic microflora.

It is a further aspect of the invention to provide a method for producing a colon-specific synthetic prodrug composition comprised of a steroid drug glycoside capable of being essentially cleaved by beta-cellobiosidase enzymatic activity of colonic microflora but not capable of being significantly hydrolyzed by endogenous enzymes produced by the mammalian host thus enabling the most significant amounts of free drug to be released in the area of the colon following cleavage of the drug glycoside by glycosidases produced by the colonic microflora.

Other objects of the invention will become apparent to those skilled in the art from the following description, taken in connection with the accompanying drawings.

FIGURE 1 is a chemical formula chart illustrating preparation and enzymatic hydrolysis of glucosides 1 and 2;

FIGURE 2 is a drawing showing a HPLC chromatogram of the cecal contents of a rat given of glucoside 1 and sacrificed six hours later;

FIGURE 3 is a drawing of graphs illustrating recovery of steroid glycoside and free steroid at various times after intragastric administration of the glycosides 1 and 2;

FIGURE 4 is a drawing of graphs illustrating recovery of steroid at various times after intragastric administration of the free steroids prednisolone and dexamethasone;

FIGURE 5 is a drawing of graphs illustrating the hydrolysis of p-nitrophenyl-glucoside at several concentrations, plotted by the Eadie-Hoffstee method to determine Vmax and Km(app).

FIGURE 6 is a drawing of graphs illustrating the hydrolysis of p-nitrophenyl-galactoside at several concentrations, plotted by the Eadie-Hoffstee method to determine Vmax and Km(app).

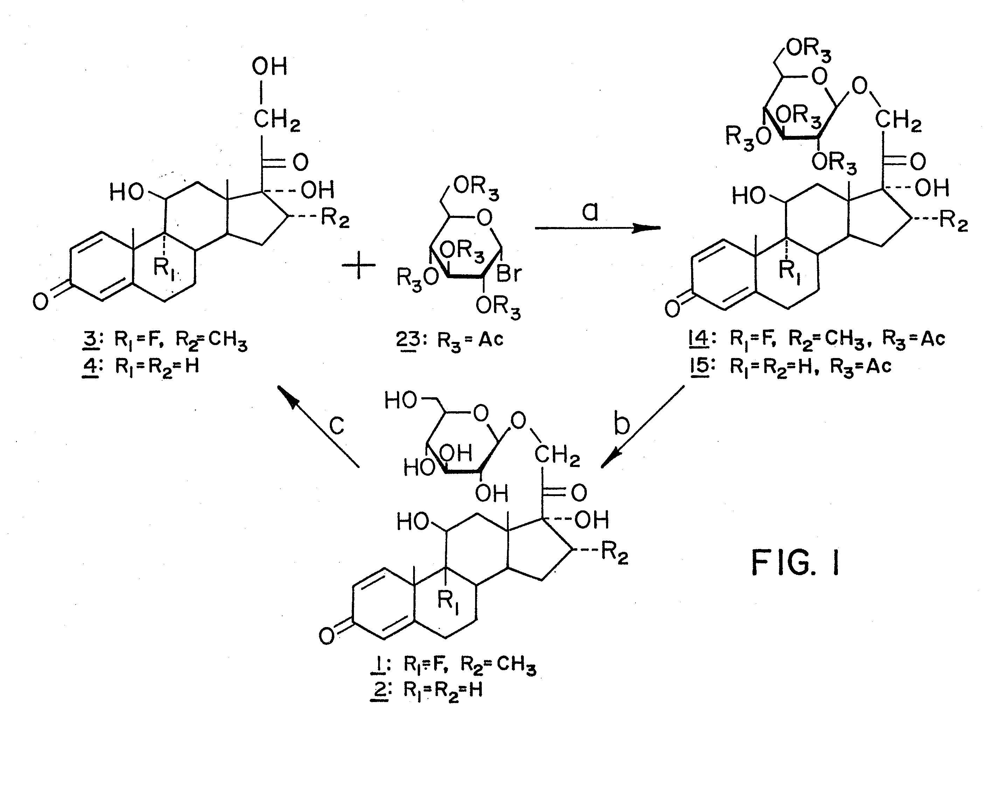

In the chart of FIGURE 1, "a" is $Ag_2CO_3$, molecular sieve, $CCl_4$; "b" is 0.01 N NaOH, MeOH; and "c" is beta-glucosidase; 1, 2, 3, 4, 23, 14 and 15 are compounds 1, 2, 3, 4, 23, 14 and 15 respectively; the letter R means radical and the letters Ac mean acetyl. The other letters, eg., O, H, F and Br denote the chemical elements oxygen, hydrogen, fluorine and bromine, respectively.

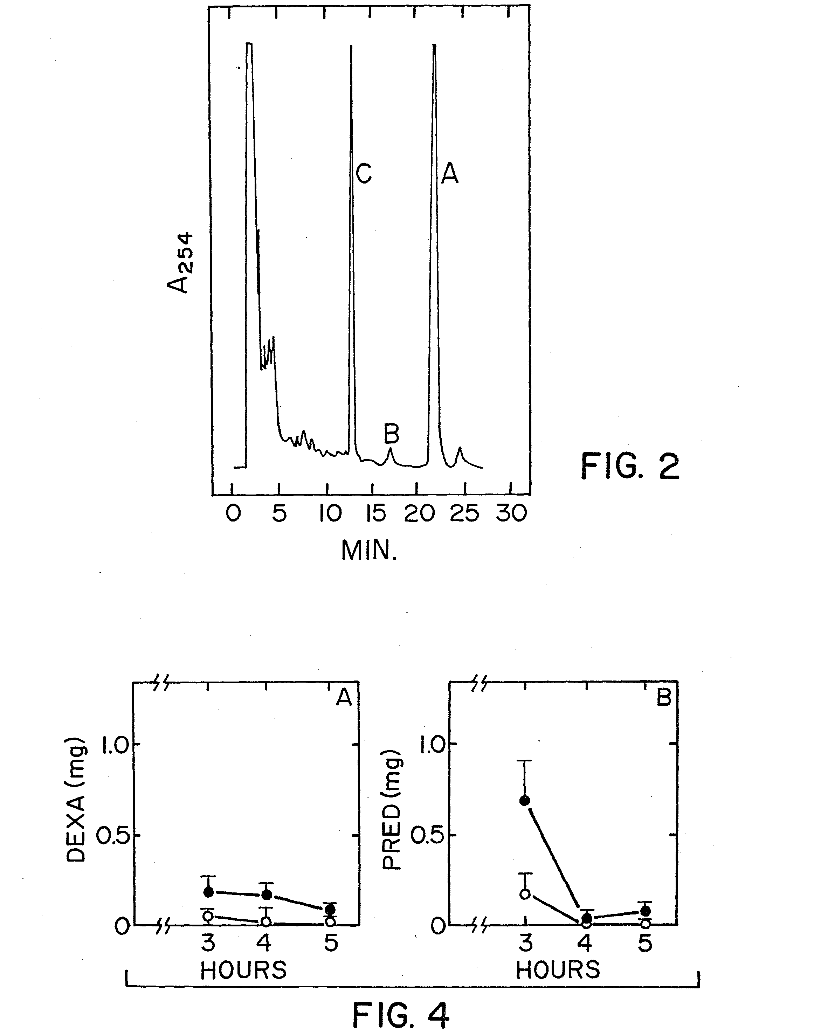

In the chromatogram of FIGURE 2, Peak A is glucoside 1; Peak B is dexamethasone, 3; and Peak C is prednisolone, 4, added as an internal standard prior to homogenization. To obtain the chromatogram, an Altex 5 micrometer Ultrasphere, C-18 column was eluted with MeOH/0.01 M $KH_2PO_4$ (56.5:43.5) at a flow rate of 1.2 mL/min.

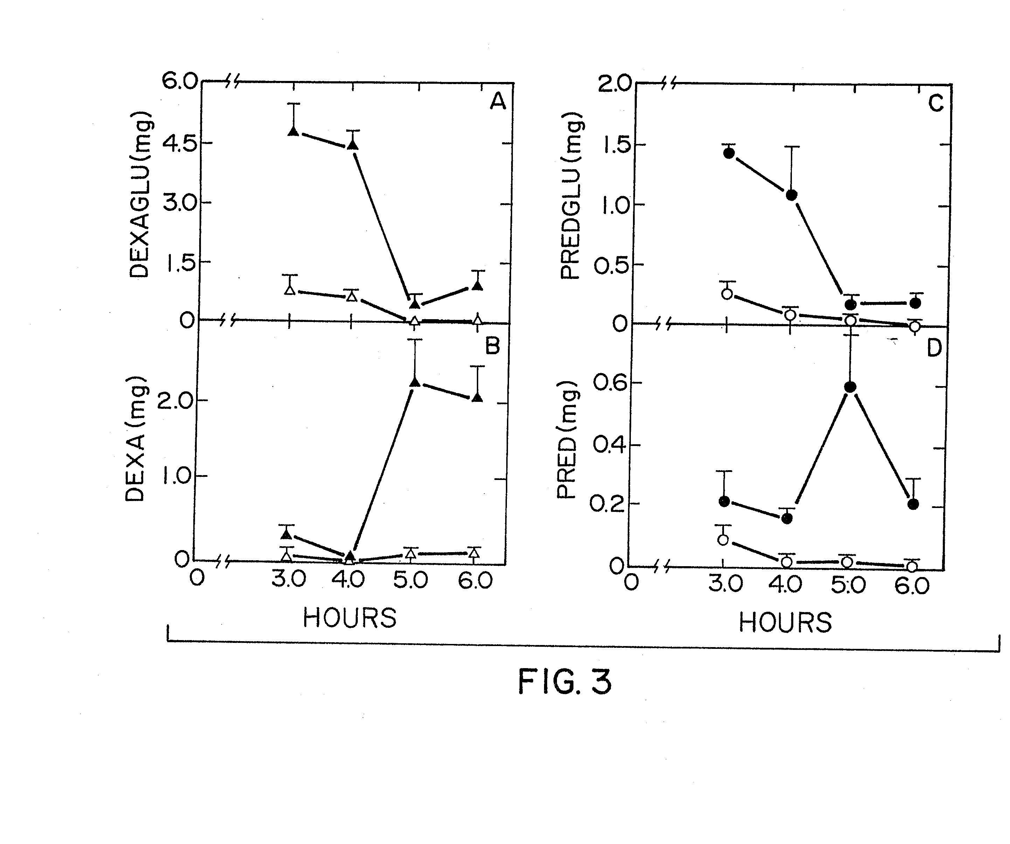

The graphs of FIGURE 3 illustrate recovery of steroid glycoside and free steroid at various times after intragastric administration of 7.5 mg of glucoside 1 (panels A and B) and glucoside 2 (panels C and D). Data are given as means ±SEM (n=4). Solid circle and triangle symbols indicate intestinal contents; and open circle and triangle symbols indicate intestinal tissues.

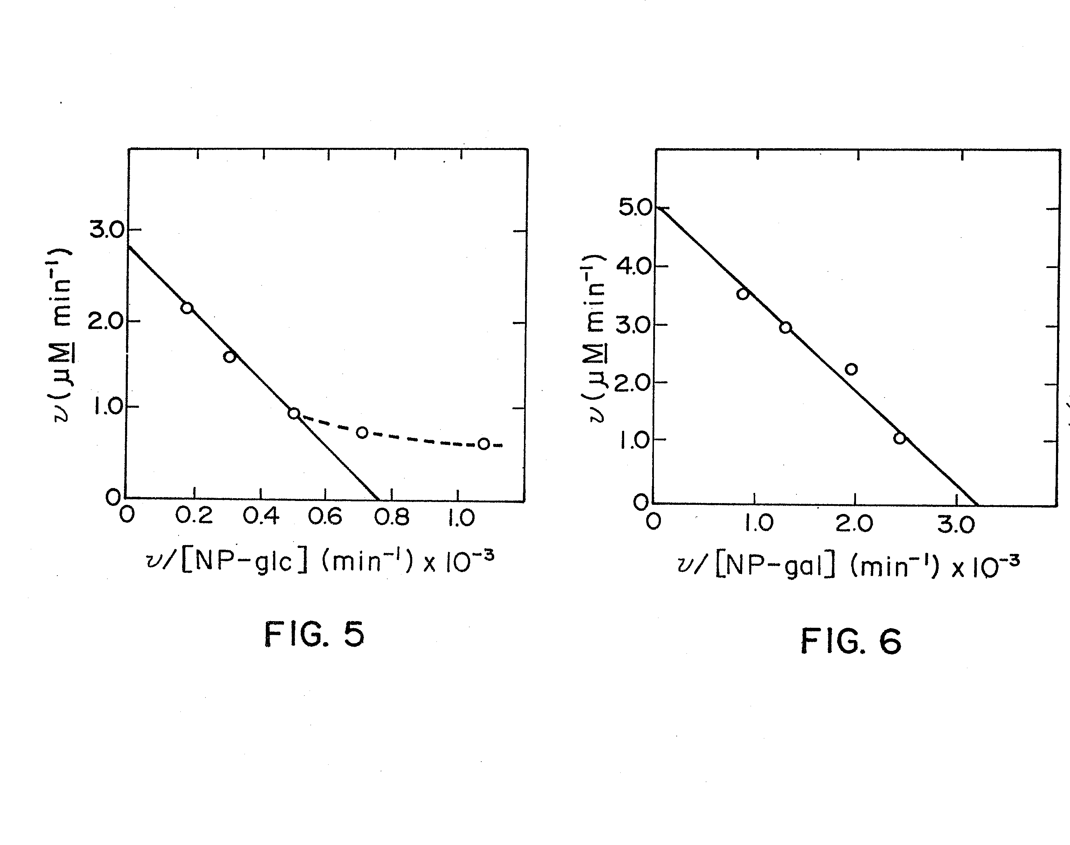

The graphs of FIGURE 4 illustrate recovery of steroid at various times after intragastric administration of the free steroids dexamethasone, 3, (5.10 mg) and prednisoone, 4, (5.25 mg). Data points are means ±SEM (n=3) from intestinal contents, shown as solid circles, and tissues, shown as open circles.

The graph of FIGURE 5 illustrates the velocity-substrate relationship for hydrolysis of p-nitrophenyl-glucoside by cecal contents at lower substrate concentrations. Hydrolysis was followed by measuring the release of p-nitrophenol spectrophotometrically at 403 nm. Eadie-Hofstee plots were used to determine the $K_{M(app)}$ and $V_{max}$. The Eadie-Hofstee plot of the relationship deviated from linearity suggesting that beta-D-glucosidase activity may be more heterogenous in nature than beta-D-galactosidase activity.

The graph of FIGURE 6 illustrates the velocity-substrate relationship for hydrolysis of p-nitrophenyl-galactoside by cecal contents. Again, hydrolysis was followed by measuring the release of p-nitrophenol spectrophotometrically at 403 nm. Eadie-Hofstee plots were used to determine the $K_{M(app)}$ and $V_{max}$. The Eadie-Hofstee plot is linear.

In the present description and claims reference will be made to several terms which are expressly defined for use herein as follows.

Colonic microflora - gastrointestinal bacteria found essentially exclusively in the colonic area of the mammalian gastrointestinal tract.

Colon-specific - essentially exclusive to the colonic area of the mammalian gastrointestinal tract.

PSI - proximal small intestine.

DSI - distal small intestine.

Endogenous enzymes - enzymes produced by the mammalian host (as opposed to enzymes produced by bacteria found within the mammalian intestine) that are secreted into the mammalian gastrointestinal tract.

Natural - produced in nature. As used herein, natural will refer to compounds produced in living organisms.

Synthetic - produced by synthesis. As used herein, synthetic will refer to compounds not produced in nature.

Simple sugar - a monosaccharide, a disaccharide or an oligosaccharide.

Aglycone - a compound having no sugar attached thereto by means of a glycosidic bond.

Glycoside - a compound which contains a sugar moiety and an aglycone moiety attached to one another by means of a glycosidic linkage.

Glycosidic link - a link between an aglycone and the reducing end of a sugar.

Drug - any chemical compound or any noninfectious biological substance, not used for its mechanical properties, which may be administered to or used on or for patients, either human or animal, as an aid in the diagnosis, treatment or prevention of disease or other abnormal condition, for the relief of pain or suffering, or to control or improve any physiological or pathological condition.

Prodrug - a latent form of an active drug with certain physicochemical properties that allow it to. reach a target organ or tissue. Once there, the active drug is formed chemically or enzymatically in situ.

Hydrophilicity - the state of being hydrophilic.

Most significantly - the most measurable amount. As used herein, the prase denotes the relative amount of aglycone released following cleavage of the glycosidic link on the prodrug when release occurs in the area of the colon as compared with release in other areas of the mammalian gastrointestinal tract.

Scientific publications and patents cited herein are expressly incorporated by reference.

The following compounds are referred to in the present description and claims:

Compound 1 is 9 alpha-Fluoro-11 beta, 17 alpha-dihydroxy-16 alpha-methyl-3, 20-dioxopregna-1, 4-dien-21-yl beta-D-glucopyranoside; it is referred to herein as "compound 1", "(1)", "1", DEXAGLU, or dexamethasone-glucoside.

Compound 2 is 11 beta, 17 alpha-Dihydroxy-3, 20-dioxopregna-1, 4-dien-21-yl beta-D-glucopyranoside; it is referred to herein as "compound 2", "(2)", "2", PREDGLU, or prednisolone-glucoside.

Compound 3 is 9 alpha-Fluoro-11 beta, 17 alpha-21 trihydroxy-16 alpha-methyl-3, 20-dioxopregna-1, 4-diene; it is referred to herein as "compound 3", "(3)", "3", "dexa", or dexamethasone.

Compound 4 is 11 beta, 17 alpha-21-Trihydroxy-3, 20-dioxpregna-1, 4-diene; it is referred to herein as "compound 4", "(4)", "4", "pred", or prednisolone.

Compound 5 is 11 beta, 17 alpha-Dihydroxy-3, 20-dioxopregna-4-en-21-yl beta-D-glucopyranoside; it is referred to herein as "compound 5", "(5)", "5", or hydrocortisone-glucoside.

Compound 6 is 11 beta, 17 alpha-21-Trihydroxy-3, 20-dioxopregna-4-ene; it is referred to herein as "compound 6", "(6)", "6", or hydrocortisone.

Compound 7 is 9 alpha-Fluoro-11 beta, 17 alpha-dihydroxy-3, 20-dioxopregna-4-en-21-yl beta-D-glucopyranoside; it is referred to herein as "compound 7", "(7)", "7", or fludrocortisone-glucoside.

Compound 8 is 9 alpha-Fluoro-11 beta, 17 alpha-21-trihydroxy-3, 20-dioxopregna-4-ene; it is referred to herein as "compound 8", "(8)", "8", or fludrocortisone.

Compound 9 is 9 alpha-Fluoro-11 beta, 17 alpha-dihydroxy-16 alpha-methyl-3, 20-dioxopregna-1, 4-dien-21-yl beta-D-galactopyranoside; it is referred to herein as "compound 9", "(9)", "9", or dexamethasone-galactoside.

Compound 10 is 11 beta, 17 alpha-Dihydroxy-3, 20-dioxopregna-4-en-21-yl beta-D-galactopyranoside; it is referred to herein as "compound 10", "(10)", "10", or prednisolone-galactoside.

Compound 11 is 11 beta, 17 alpha-Dihydroxy-3, 20-dioxopregna-4-en-21-yl beta-D-galactopyranoside; it is referred to herein as "compound 11", "(11)", "11", or hydrocortisone-galactoside.

Compound 12 is 9 alpha-Fluoro-11 beta, 17 alpha-dihydroxy-3, 20-dioxopregna-4-en-21-yl beta-D-galactopyranoside; it is referred to herein as "compound 12", "(12)", "_12_", or fludrocortisone-galactoside.

Compound 13 is 11 beta, 17 alpha-Dihydroxy-3, 20-dioxopregna-1, 4-diene-21-yl beta-D-cellbioside; it is referred to herein as "compound 13", "(13)", "_13_", or prednisolone-cellobioside.

Compound 14 is 9 alpha-Fluoro-11 beta, 17 alpha-dihydroxy-16 alpha-methyl-3, 20 dioxo pregna-1, 4-dien-21-yl 2', 3', 4', 6'-tetra-_O_-acetyl-beta-D-glucopyranoside; it is referred to herein as "compound 14", "(14)", "_14_", or DEXATAGLU.

Compound 15 is 11 beta, 17 alpha-Dihydroxy-3, 20-dioxopregna-1, 4-dien-21-yl 2', 3', 4', 6'-tetra-_O_-acetyl-beta-D-glucoside; it is referred to herein as "compound 15", "(15)", "_15_", or PREDTAGLU.

Compound 16 is 11 beta, 17 alpha-Dihydroxy-3, 20-dioxopregna-4-en-21-yl 2', 3', 4', 6'-tetra-_O_-acetyl-beta-D-glucopyranoside; it is referred to herein as "compound 16", "(16)", "_16_", or hydrocortisone tetraacetyl glucoside.

Compound 17 is 9 alpha-Fluoro-11 beta, 17 alpha dihydroxy-3, 20-dioxopregna-4-en-21-yl 2', 3', 4', 6'-tetra-_O_-acetyl-beta-D-glucopyranoside; it is referred to herein as "compound 17", "(17)", "_17_", or fludrocortisone tetraacetyl glucoside.

Compound 18 is 9 alpha-Fluoro-11 beta, 17 alpha-dihydroxy-16 alpha-methyl-3, 20-dioxopregna-1, 4-dien-21-yl 2', 3', 4', 6'-tetra-_O_-acetyl-beta-D-galactopyranoside; it is referred to herein as "compound 18", "(18)", "_18_", or dexamethasone tetraacetyl galactoside.

Compound 19 is 11 beta, 17 alpha-Dihydroxy-3, 20-dioxopregna-1, 4-dien-21-yl 2', 3', 4', 6'-tetra-_O_-acetyl-beta-D-galactopyranoside; it is referred to here as "compound 19", "(19)", "19", or prednisolone tetraacetyl galactoside.

Compound 20 is 11 beta, 17 alpha-Dihydroxy-3, 20-dioxopregna-4-en-21-yl 2', 3', 4', 6'-tetra-_O_-acetyl-beta-D-galactopyranoside; it is referred to herein as "compound 20", "(20)", "20", or hydrocortisone tetraacetyl galactoside.

Compound 21 is 9 alpha-Fluoro-11 beta, 17 alpha dihydroxy-3, 20-dioxopregna-4-en-21-yl 2', 3', 4', 6'-tetra-_O_-acetyl-beta-D-galactopyranoside; it is referred to herein as "compound 21", "(21)", "21", or fludrocortisone tetraacetyl galactoside.

Compound 22 is 11 beta, 17 alpha-Dihydroxy-3, 20-dioxopregna-1, 4-dien-21-yl hepta-_O_-acetyl-beta-D-cellobioside; it is referred to herein as "compound 22", "(22)", "22", or prednisolone heptaacetyl cellobioside.

Compound 23 is 2, 3, 4, 6-tetra-_O_-acetyl-1-bromo-alpha-D-glucopyranose; it is referred to herein as "compound 23", "(23)", "23", or tetraacetyl-1-bromo-glucose.

Compound 24 is 2,3,4,6-tetra-_O_-acetyl-1-bromo-alpha-D-galactopyranose; it is referred to herein as "compound 24", "(24)", "24", or tetraacetyl-1-bromo-galactose.

Compound 25 is hepta-_O_-acetyl-1-bromo-alpha-D-cellobiose; it is referred to herein as "compound 25", "(25)", "25", or heptaacetyl-1-bromo-alpha-cellobiose.

All steroids, 2,3,4,6-tetra-_O_-acetyl-1-bromo-alpha-D-glucopyranose, 2,3,4,6-tetra-_O_-acetyl-1-bromo-alpha-D-galactopyranose, _p_-nitrophenyl substrates, and _p_-nitrophenol were purchased from Sigma Chemical Co. Octa-_O_-acetyl-alpha-D-cellobiose was obtained from Aldrich Chemical Co. and the 31% hydrobromic acid in acetic acid was purchased from Eastman Kodak Co.

Preparative methods and assays utilized in the disclosure of the present invention include:

PREPARATIVE METHODS

SOLVENTS

All solvents were redistilled and dried over molecular sieves, 4 angstrom, 4-8 mesh (Aldrich Chemical Co.). All solvent evaporations were performed with a rotary evaporator with water aspirator reduced pressure. Melting points were obtained on a Buchi melting point apparatus and are uncorrected. UV spectra were determined on a Cary 210 spectrometer. IR spectra were determined on a Perkin Elmer Model 137 spectrometer. $^1$H NMR spectra were determined on either the UCB 200 or the UCB 250 (home-made 200 and 250-MHz Fourier transform devices located in the College of Chemistry, University of California, Berkeley) and were recorded in dimethyl-$\underline{d}_6$ sulfoxide; they are expressed in parts per million (delta) downfield from $Me_4Si$ with coupling constants (J) expressed in hertz. Elemental analyses were performed by the Analytical Laboratory, College of Chemistry, University of California, Berkeley. Analyses were within ± 0.4% of theoretical values except where noted.

CHROMATOGRAPHY

High-pressure liquid chromatography (HPLC) was performed on an Altex analytical system consisting of two model 110A pumps, a model 160 UV detector, a model 420 microprocessor/programmer and a stainless steel column (4.6 x 25 cm, 5 micrometer Ultrasphere C-18). A flow rate of 1.2 mL/min was used, with absorbance monitoring at 254 nm. The solvent system for all separations was MeOH/0.01 $\underline{M}$ $KH_2PO_4$ (56.5:43.5). Low-pressure preparative chromatography (flash chromatography, J.T. Baker Chem. Co.) was performed using either a 3.7 x 22 cm column of 40 micrometer RP-18 with MeOH/water (68:32) as eluent or a 3.0 x 18 cm column of 40 micrometer silica gel with $CHCl_3$/95% EtOH (65:35) as eluent. TLC was performed on aluminum-backed plates of silica gel 60 (E. Merck Co.). Steroids and their glycosides were identified by spraying the developed plates with toluenesulfonic acid/95% EtOH (20:80, w/v) and heating for 10 min at 110°C.

SYNTHESIS

COMPOUND 15: PREDTAGLU

11 beta, 17 alpha-Dihydroxy-3, 20-dioxopregna-1, 4-dien-21-yl 2', 3', 4', 6'-tetra-O-acetyl-beta-D-glucoside.

PREDNISOLONE 4, (1.20 g, 3.33 mmoles) was dissolved in dry $CHCl_3$ (30 ml) and added to dry, boiling $CHCl_3$ (200 ml) over 4 angstrom molecular sieves in a 500 ml round bottom flask. After 10-20 ml had been distilled, freshly prepared (See McCloskey, C.M. and Colman, G.H., in "Organic Synthesis" Coll. Vol. 3, Wiley, New York, N.Y. (1955) pp 434-435) silver carbonate (3.90 g, 14.1 mmoles) was added to the flask. Then a solution of 2,3,4,6-tetra-O-acetyl-1-bromo-alpha-D-glucopyranose (23, 3.50 g, 8.50 mmoles) in dry $CHCl_3$ (100 ml) was added dropwise from an addition funnel. The reaction mixture was protected from light and stirred throughout. The addition of bromosugar took approximately 1 hr., and the solvent was distilled continuously during this time. Distillation was continued for an additional hour after all the bromosugar had been added; volume was maintained by the addition of dry $CHCl_3$. The solution was filtered, washed with cold, saturated NaCl, dried with sodium sulfate, and the solvent removed. The oily residue was dissolved in several milliliters of methanol and purified by flash chromatography on RP-18. The appropriate fractions were collected and the solvent removed. Crystallization of PREDTAGLU from methanol/water yielded 0.87 g (38%); MP: 119-121°C; TLC: $R_f$ 0.36 (ethyl acetate/isooctane 9:1); UV lambda(max): 242 nm (epsilon 13500); IR(KBr): 3450 (OH), 1760 (acetyl), 1650 (C=O), 1190 (acetyl), 896 cm$^{-1}$; $^1$H-NMR: delta 0.77 (s, 3H, C-18), 1.45 (s, 3H, C-19), 1.99 (s, 9H, C-2',3',4' acetyl), 2.08 (s, 3H, C-6' acetyl), 4.20 (d, 1H, C-1', J=8), 4.58 (AB, q, 2H, C-21, J=18, 5.92 (s, 1H, C-4), 6.15 (d, 1H, C-2, J=11), 7.40 (d, 1H, C-1, J=11). Anal. ($C_{35}H_{46}O_{14}$) C, H.

## COMPOUND 14: DEXATAGLU

9 alpha-Fluoro-11 beta, 17 alpha-dihydroxy-16 alpha-methyl-3, 20 dioxopregna-1, 4-dien-21-yl 2', 3', 4', 6'-tetra-O-acetyl-beta-D-glucopyranoside.

DEXATAGLU, 14, was prepared from dexamethasone, 3, as described for PREDTAGLU, 15, from prednisolone, 4. Crystallization of DEXATAGLU from methanol/water yielded 0.55 gm (25%); MP: 119.5-121°C; TLC: $R_f$ 0.45 (ethyl acetate/isooctane, 9:1); UV lambda(max): 239 nm (epsilon 14300), IR(KBr): 3450 (OH), 1760 (acetyl), 1650 (C=O), 1190 (acetyl), 896 cm$^{-1}$; $^1$H NMR: delta 0.76 (d, 3H, C-16 alpha $CH_3$, J=6), 0.88 (s, 3H, C-18), 1.49 (s, 3H, C-19), 2.00 (s, 9H, C-2', 3', 4', acetyl), 2.09 (s, 3H, C-6' acetyl), 4.18 (d, 1H, C-1', J=8), 4.57 (AB q, 2H, C-21, J=18), 6.02 (s, 1H, C-4), 6.23 (d, 1H, C-1, J=11), 7.33 (d, 1H, C-2 J=11). Anal. Calc. for $C_{36}H_{47}O_{14}F$: C, 59.75; H, 6.63. Found: C, 59.08 H, 6.54.

## COMPOUND 2: PREDNISOLONE-GLUCOSIDE

11 beta, 17 alpha-Dihydroxy-3, 20-dioxopregna-1, 4-dien-21-yl beta-D-glucopyranoside.

PREDTAGLU, 15, (0.20 g, 0.38 mmoles) was dissolved in methanol (10 ml) and benzene (5.0 ml). 0.04 $\underline{N}$ NaOH in methanol (5.0 ml) was then added. The reaction was run under $N_2$ at room temperature with stirring. After thirty min., several drops of acetic acid were added to neutralize the solution. The solvent was removed under reduced pressure and the residue purified by flash chromatography on silica gel. The appropriate fractions were combined, the solvent removed under reduced pressure, and the residue dissolved in a $\underline{tert}$-butyl alcohol/water (15 ml., 1:1) solution. This solution was frozen and the solvent removed by lyophilization. Yield was 0.11 g (73%); TLC: $R_f$ 0.58 (chloroform/95% ethanol, 3:2); UV lambda(max): 242 nm (epsilon 13200); IR(KBr): 3450 (-OH), 1650 (C=O), 896 cm$^{-1}$; $^1$H-NMR: delta 0.77 (s, 3H, C-18), 1.45 (s, 3H, C-19), 4.20 (d, 1H, C-1', J=8), 4.58 (AB q, 2H, C-21, J=18), 5.92 (s, 1H, C-4), 6.15 (d, 1H, C-1, J=11), 7.40 (d, 1H, C-2, J=11). Anal. Calc. for $C_{27}H_{38}O_{10} \cdot H_2O$: C, 60.00; H, 7.41. Found: C, 59.94; H, 7.64.

COMPOUND 1: DEXAMETHASONE-GLUCOSIDE

9 alpha-Fluoro-11 beta, 17 alpha-dihydroxy-16 alpha-methyl-3, 20-dioxopregna-1, 4-dien-21-yl beta-D-glucopyranoside.

Compound 1 was prepared from DEXATAGLU, $\underline{14}$, as described for prednisolone glucoside, $\underline{2}$, from PREDTAGLU, $\underline{15}$. Yield was 0.12 gm (75%); TLC: $R_f$ 0.51 (chloroform/95% ethanol, 3:2); UV lambda(max): 239 nm (epsilon 14500); IR(KBr): 3450 (-OH), 1650 (C=O), 896 cm$^{-1}$; $^1$H NMR: delta 0.78 (d, 3H, C-16 alpha methyl, J=7), 0.88 (s, 3H, C-18), 1.49 (s, 3H, C-19), 4.17 (d, 1H, C-1'; J=8), 4.57 (AB q, 2H, C-21, J=18), 6.03 (s, 1H, C-4), 6.23 (d, 1H, C-1, J=11), 7.35 (d, 1H, C-2, J=11). Anal. Calc. for $C_{28}H_{39}O_{12}F \cdot H_2O$: C, 58.43; H, 7.65. Found: C, 58.58; H, 7.36.

COMPOUND 18: DEXAMETHASONE TETRAACETYL GALACTOSIDE

9 alpha-Fluoro-11 beta, 17 alpha-dihydroxy-16 alpha-methyl-3,20-dioxopregna-1,4-dien-21-yl 2',3',4',6'-tetra-$\underline{O}$-acetyl-beta-D-galactopyranoside.

Compound 18 was prepared from dexamethasone, $\underline{3}$, as described for PREDTAGLU, $\underline{15}$, from steroid $\underline{4}$, prednisolone. Crystallization of $\underline{18}$ from MeOH/water yielded 0.56 g (26%); MP: 133-135°C; TLC: $R_f$ 0.39 (ethyl acetate/isooctane, 9:1); UV lambda(max): 239 nm (epsilon 15400); IR(KBr): 3450 (OH), 1750 (OAc), 1660 (C=O), 1620 (C=C), 1240 (OAc), 985, 960, 895 cm$^{-1}$; $^1$H NMR delta 0.78 (d, 3H, C-16 CH$_3$), 0.88 (s, 3H, C-18), 1.49 (s, 3H, C-19), 1.93 (s, 3H, C-4' OAc), 2.00 (s, 3H, C-3' OAc), 2.06 (s, 3H, C-2' OAc), 2.12 (s, 3H, C-6' OAc), 4.18 (d, 1H, C-1', J=7.2), 4.52 (ABq, 2H, C-21, J=18.3), 6.01 (s, 1H, C-2, J=11), 7.32 (d, 1H, C-1, J=10). Anal. (C$_{36}$H$_{47}$O$_{14}$F) C, H.

COMPOUND 19: PREDNISOLONE TETRAACETYL GALACTOSIDE

11 beta, 17 alpha-Dihydroxy-3,20-dioxopregna-1,4-dien-21-yl 2',3',4',6'-tetra-$\underline{O}$-acetyl-beta-D-galactopyranoside.

Compound 19 was prepared from prednisolone ($\underline{4}$) as described for PREDTAGLU, $\underline{15}$, from steroid $\underline{4}$, prednisolone. Crystallization of $\underline{19}$ from MeOH/water yielded 0.82 g (37%); MP: 134-136°C; TLC: $R_f$ 0.39 (ethyl acetate/isooctane, 9:1; UV lambda(max): 242 nm (epsilon 14700); IR(KBr): 3500 (OH), 1760 (OAc), 1650 (C=O), 1620 (C=C), 1240 (OAc), 900 cm$^{-1}$; $^1$H NMR delta 0.78 (s, 3H, C-18), 1.38 (s, 3H, C-19), 1.91 (s, 3H, C-4' OAc), 1.99 (s, 3H, C-3' OAc), 2.03 (s, 3H, C-2' OAc), 2.09 (s, 3H, C-6' OAc), 4.23 (d, 1H, C-1', J=7.2), 4.49 (ABq, 2H, C-21, J=18), 5.92 (s, 1H, C-4), 6.15 (d, 1H, C-1, J=11), 7.40 (d, 1H, C-2, J=11). Anal. (C$_{35}$H$_{46}$O$_{14}$) Calcd: C, 60.78; H, 6.66. Found: C, 59.83; H, 6.89.

COMPOUND 16: HYDROCORTISONE TETRAACETYL GLUCOSIDE

11 beta, 17 alpha-Dihydroxy-3,20-dioxopregna-4-en-21-yl 2',3',4',6'-tetra-O-acetyl-beta-D-glucopyranoside.

Compound 16 was prepared from hydrocortisone (6) and bromo sugar 23 as described for glucoside 15 from steroid 4 and bromo sugar 23. Crystallization of 16 from MeOH/water yielded 0.52 g (23%); MP: 120.5-122°C; TLC: $R_f$ 0.39 (ethyl acetate/isooctane, 9:1); UV lambda(max): 242 nm (epsilon 15700); IR(KBr): 3450 (OH), 1760 (OAc), 1645 (C=O), 1610 (C=C), 1240 (OAc), 950, 908, 875 cm$^{-1}$; $^1$H NMR delta 0.80 (s, 3H, C-18, 1.40 (s, 3H, C-19), 1.96 (s, 3H C-4' OAc), 1.99 (s, 3H, C-3' OAc), 2.04 (s, 3H, C-2' OAc), 2.05 (s, 3H, C-6' OAc), 4.20 (d, 1H, C-1', J=7.5), 4.60 (ABq, 2H, C-21, J=18), 5.60 (s, 1H, C-4. Anal. ($C_{35}H_{48}O_{14}$) H; C: calcd, 60.69; found 60.02.

COMPOUND 20: HYDROCORTISONE TETRAACETYL GALACTOSIDE

11 beta, 17 alpha-Dihydroxy-3,20-dioxopregna-4-en-21-yl 2',3',4',6'-tetra-O-acetyl-beta-D-galactopyranoside.

Compound 20 was prepared from steroid 6 and bromo sugar 24 as described for glucoside 15 from steroid 4. Crystallization of 20 from MeOH/water yielded 0.57 g (26%); MP: 122-124°C; TLC: $R_f$ 0.42 (ethyl acetate/isooctane); UV lambda(max): 242 nm (epsilon 16700); IR(KBr): 3450 (OH), 1760 (OAc), 1660 (C=O), 1620 (C=C), 1230 (OAc), 950, 908, 896 cm$^{-1}$; $^1$H NMR delta 0.80 (s, 3H, C-18), 1.42 (s, 3H, C-19, 1.92 (s, 3H, C-4' OAc), 1.98 (s, 3H, C-3' OAc), 2.04 (s, 3H, C-2' OAc), 2.09 (s, 3H, C-6' OAc), 4.24 (d, 1H, C-1', J=7.6), 4.58 (ABq, 2H;, C-21, J=18), 5.60 (s, 1H, C-4). Anal. ($C_{35}H_{48}O_{14}$) H, C: calcd, 60.69; found, 60.27.

COMPOUND 17: FLUDROCORTISONE TETRAACETYL GLUCOSIDE

9 alpha-Fluoro-11 beta, 17 alpha-dihdroxy-3, 20-dioxopregna-4-en-21-yl 2',3',4',6'-tetra-O-acetyl-beta-D-glucopyranoside.

Compound 17 was prepared from fludrocortisone 8 as described for glucoside 15 from steroid 4. Crystallization of 17 from MeOH/water yielded 0.48 g (23%); MP: 124-125°C; TLC: $R_f$ 0.39 (ethyl acetate/isooctane); UV lambda(max): 239 nm (epsilon 17500); IR(KBr): 3450 (OH), 1750 (OAc), 1660 (C=O), 1625 (C=C), 1250 (OAc), 898 cm$^{-1}$; $^1$H NMR delta 0.76 (s, 3H, C-18), 1.49 (s, 3H, C-19), 1.95 (s, 3H, C-4' OAc), 2.00 (s, 6H, C-2',3' OAc), 2.04 (s, 3H, C-6' OAc), 4.25 (d, 1H, C-1', J-7.8), 4.45 (ABq, 2H, C-21, J=18, 5.75 (s, 1H, C-4). Anal. ($C_{35}H_{47}O_{14}F$) C, H.

COMPOUND 21: FLUDROCORTISONE TETRAACETYL GALACTOSIDE

9 alpha-Fluoro-11 beta, 17 alpha dihydroxy-3, 20-dioxopregna-4-en-21-yl 2', 3', 4', 6'-tetra-O-acetyl-beta-D-galactopyranoside.

Compound 21 was prepared from steroid 8 and bromo sugar 24 as described for glucoside 15 from steroid 4 and bromo sugar 23. Crystallization of 21 from MeOH/water yielded 0.45 g (23%); MP: 130-132°C; TLC: $R_f$ 0.40 (ethyl acetate/isooctane); UV lambda(max): 239 nm (epsilon 17100); IR(KBr): 3500 (OH), 1780 (OAc), 1660 (C=O), 1620 (C=C), 1250 (OAc) cm$^{-1}$; $^1$H NMR: delta 0.78 (s, 3H, C-18), 1.48 (s, 3H, C-19), 1.92 (s, 3H, C-4' OAc), 1.99 (s, 3H, C-3' OAc), 2.03 (s, 3H, C-2' OAc), 2.09 (s, 3H, C-6' OAc), 4.25 (d, 1H, C-1', J=8), 4.53 (ABq, 2H, C-21, J=18), 5.73 (s, 1H, C-4). Anal. ($C_{35}H_{47}O_{14}F$) C, H.

COMPOUND 25: HEPTAACETYL-1-BROMO-ALPHA CELLOBIOSE

Hepta-O-acetyl-1-bromo-alpha-D-cellobiose, was prepared according to published procedures. See Bollenback, G.N., Long, J.W., Benjamin, D.G., Lindquist, J.A., J. Am. Chem. Soc., 77:3310 (1955); Freudenberg, K., Nagai, W., Ann., 494:63 (1932).

Octa-O-acetyl-alpha-D-cellobiose (7.5 g, 11.1 mmol) was dissolved in 31% HBr in acetic acid (35 mL). The mixture was stirred at 4 degrees C for 24 h. Then icecold water (5 mL) was added, followed by $CHCl_3$ (10 mL). The organic phase was then washed several times with cold saturated NaCl solution, and dried ($Na_2SO_4$). Bromo sugar 25 was crystallized by addition of dry ethyl ether to yield 4.68 g (61%); MP: 181-182°C (lit. MP 183°C, (See Freudenberg and Nagai, supra), [alpha]$_D^{27}$ +93.5 (c 5.4 $CHCl_3$), (lit. [alpha]$_D^{20}$ +95.8. See Haynes, L.J., Newth, F.H., Adv. Carbohydr. Chem., 10:213 (1955).

### COMPOUND 22: PREDNISOLONE HEPTAACETYL CELLOBIOSIDE

11 beta, 17 alpha-Dihydroxy-3,20-dioxopregna-1,4-dien-21-yl Hepta-O-acetyl-beta-D-cellobioside.

Compound 22 was prepared from steroid 4 (0.6 g, 1.6 mmol) and bromo sugar 25 (3.7 g, 5.2 mmol) as described for the preparation of glucoside 15 from steroid 4 and bromo sugar 23. Crystallization of 22 from MeOH/water yielded 0.42 g (25%); MP: 135-136°C; TLC: $R_f$ 0.39 (ethyl acetate/isooctane); UV lambda(max): 242 nm (epsilon 16900); IR(KBr): 3500 (OH), 1750 (OAc), 1660 (C=O), 1620 (C=C), 1230 (OAc), 912, 870, 782 cm$^{-1}$; $^1$H NMR delta 0.78 (s, 3H, C-18), 1.42 (s, 3H, C-19), 1.91 (s, 3H, OAc), 1.97 (s, 6H, OAc), 1.99 (s, 3H, OAc), 2.01 (s, 6H, OAc), 2.07 (s, 3H, OAc), 4.28 (d, 1H, C-1', J=7.6), 4.49 (ABq, 2H, C-21, J=18), 5.92 (s, 1H, C-4), 6.15 (d, 1H, C-2, J=11), 7.40 (d, 1H, C-1, J=10. Anal. ($C_{47}H_{62}O_{22}$) C, H.

### COMPOUND 5: HYDROCORTISONE-GLUCOSIDE

11 beta, 17 alpha-Dihydroxy-3,20-dioxopregna-4-en-21-yl beta-D-glucopyranoside.

To prepare glucoside 5, acetyl glucoside 16 (0.2 g, 0.32 mmol) was dissolved in MeOH (10 mL) and benzene (5 mL). NaOH in MeOH (0.04 N, 5.0 mL) was then added. The reaction was run under $N_2$ at room temperature with stirring. After 45 min, several drops of acetic acid were added to neutralize the solution. The solvent was removed and the residue was purified by flash chromatography on silica gel. Purified glucoside 16 was then dissolved in tert-butyl alcohol/water (15 mL, 1:1). This solution was frozen, and the solvent was removed by lyophilization to yield 0.12 mg (77%); $R_f$ 0.49 (CHCl3/95% EtOH, 65:35); UV lambda(max): 242 nm (epsilon 15800); IR(KBr): 3450 (OH), 1650 (C=O), 1620 (C=C), 945, 910, 868 cm$^{-1}$; $^1$H NMR delta 0.76 (s, 3H, C-18), 1.36 (s, 3H, C-19), 4.17 (d, 1H, C-1', J=7.7), 4.57 (ABq, 2H, C-21, J=18.2), 5.56 (s, 1H, C-4). Anal. ($C_{27}H_{40}O_{10}H_2O$) C, H.

COMPOUND 7: FLUDROCORTISONE-GLUCOSIDE

9 alpha-Fluoro-11 beta,17 alpha-dihydroxy-3, 20-dioxopregna-4-en-21-yl beta-D-glucopyranoside.

Compound 7 was prepared from acetyl glucoside 17 as described for glucoside 5 from acetyl glycoside 16. After lyophilization, 0.11 g (71%) of glucoside 7 was obtained; TLC: $R_f$ 0.50 (CHCl3/95% EtOH, 7:3); UV lambda(max): 239 nm (epsilon 17800); IR(KBr): 3450 (OH), 1650 (C=O), 1620 (C=C), 938, 895 cm$^{-1}$; $^1$H NMR delta 0.78 (s, 3H, C-18), 1.49 (s, 3H, C-19), 4.19 (d, 1H, C-1', J=7.6, 4.57 (ABq, 2H, C-21, J=18), 5.70 (s, 1H, C-4). Anal. ($C_{27}H_{40}O_{10}F \cdot H_2O$) H. C: Calcd, 57.96; found, 58.53.

COMPOUND 9: DEXAMETHASONE-GALACTOSIDE

9 alpha-Fluoro-11 beta, 17 alpha-dihydroxy-16 alpha-methyl-3,20-dioxopregna-1,4-dien-21-yl beta-D-galactopyranoside.

Compound 9 was prepared from acetyl galactoside 18 as described for glucoside 5 from acetyl glucoside 16. After lyophilization, 95 mg (60%) of galactoside 9 was obtained; TLC: $R_f$ 0.46

(CHCl$_3$/95% EtOH, 7:3); UV lambda(max): 239 nm (epsilon 14400); IR(KBr): 3450 (OH), 1680 (C=O), 1625 (C=C), 990, 891 cm$^{-1}$; $^1$H NMR delta 0.76 (d, 3H, C-16 -CH$_3$, J=7), 0.85 (s, 3H, C-18), 1.50 (s, 3H, C-19), 4.19 (d, 1H, C-1', J=7.8), 4.58 (ABq, 2h, C-21, J=18), 6.01 (s, 1H, C-4), 6.24 (d, 1H, C-1, J=10.1), 7.39 (d, 1H, C-2, J=10.2). Anal. (C$_{28}$H$_{39}$O$_{10}$F · H$_2$O) calcd: C, 60.65; H, 7.17. Found: C, 60.44; H, 6.98.

COMPOUND 10: PREDNISOLONE-GALACTOSIDE

11 beta, 17 alpha-dihydroxy-3,20-dioxopregna-1,4-dien-21-yl beta-D-galactopyranoside.

Compound 10 was prepared from acetyl galactoside 19 as described for glucoside 5 from acetyl glucoside 16. After lyophilization, 0.13 g (83%) of galactoside 10 was obtained; TLC: R$_f$ 0.39 (CHCl$_3$/95% EtOH, 7:3); UV lambda(max): 242 nm (epsilon 14500);IR(KBr): 3450 (OH), 1650 (C=O). 1615 (C=C), 899 cm$^{-1}$; $^1$H NMR delta 0.78 (s, 3H, C-18), 1.39 (s, 3H, C-19), 4.13 (d, 1H, C-1', J=7.3), 4.53 (ABq, 2H, C-21, J=18.1), 6.16 (d, 1H, C-1, J=10.1), 7.33 (d, 1H, C-2, J=10.1). Anal. (C$_{27}$H$_{38}$O$_{10}$ · H$_2$O) C, H.

COMPOUND 11: HYDROCORTISONE-GALACTOSIDE

11 beta, 17 alpha-Dihydroxy-3,20-dioxopregna-4-en-21-yl beta-D-galactopyranoside.

Compound 11 was prepared from acetyl galactoside 20 as described for glucoside 5 from acetyl glucoside 16. After lyophilization, 95 mg (60%) of compound 11 was obtained; TLC: R$_f$ 0.42 (CHCl$_3$/95% EtOH, 7:3); UV lambda(max): 242 nm (epsilon 15700); IR(KBr): 3450 (OH), 1660 (C=O), 1620 (C=C), 1080, 1033, 930, 896, 870 cm$^{-1}$; $^1$H NMR delta 0.79 (s, 3h, C-18), 1.42 (s, 3H, C-19), 4.17 (d, 1H, C-1', J=7.6), 4.54 (ABq, 2H, C-21, J=18), 5.58 (s, 1H, C-4). Anal. (C$_{27}$H$_{40}$O$_{10}$ · H$_2$O) C, H.

COMPOUND 12: FLUDROCORTISONE-GALACTOSIDE

9 alpha-Fluoro-11 beta, 17 alpha-dihydroxy-3, 20-dioxopregna-4-en-21-yl beta-D-galactopyranoside.

Compound 12 was prepared from acetyl galactoside 21 as described for glucoside 5 from acetyl glucoside 16. After lyophilization, 0.11 g (71%) of compound 12 was obtained; TLC: $R_f$ 0.49 (CHCl$_3$/95% EtOH, 7:3); UV lambda(max): 239 nm (epsilon 17700); IR(KBr): 3450 (OH), 1660 (C=O), 1620 (C=C), 1080, 1033, 930, 896, 870 cm$^{-1}$; $^1$H NMR delta 0.78 (s, 3H, C-18), 1.50 (s, 3H, C-19), 4.20 (d, 1H, C-1', J=7.8), 4.55 (ABq, 2H, C-21, J=18), 5.70 (s, 1H, C-4). Anal. ($C_{27}H_{39}O_{10}F \cdot H_2O$) C, H.

COMPOUND 13: PREDNISOLONE-CELLOBIOSIDE

11 beta, 17 alpha-Dihydroxy-3,20-dioxopregna-1,4-dien-21-yl beta-D-cellobioside.

Compound 13 was prepared from acetyl cellobioside 22 as described for glucoside 5 from acetyl glucoside 16. After lyophilization, 86 mg (60%) of compound 13 was obtained; TLC: $R_f$ 0.33 (CHCl$_3$/95% EtOH, 7:3); UV lambda(max): 242 nm (epsilon 15500); IR(KBr): 3450 (OH), 1680 (C=O), 1620 (C=C), 1170, 950, 898, 875 cm$^{-1}$; $^1$H NMR delta 0.75 (s, 3H, C-18), 1.28 (s, 3H, C-19), 4.15 (d, 1H, C-1', J=7.6), 4.51 (ABq, 2H, C-21, J=18), 5.91 (s, 1H, C-4), 6.20 (d, 1H, C-1, J=10.2), 7.30 (d, 1H, C-2, J=10.1). Anal. ($C_{33}H_{48}O_{15} \cdot H_2O$) C, H.

IN VIVO TESTING

Male, Sprague-Dawley rats (ca 250 g) were maintained on laboratory rat chow and water ad lib. These rats were fasted overnight (16 hr.) prior to administration of glucoside or free steroid. Water bottles were removed from the cages at least 30 min prior to drug administration. Prednisolone glucoside, 2, (7.5 mg) or dexamethasone glucoside, 1, (7.5 mg) was administered by gastric intubation as a solution (0.5

0123485

-24-

mL) of water/95% ethanol (3:1). Prednisolone, 4, (5.25 mg) or dexamethasone, 3, (5.1 mg) was administered as a solution (0.5 mL) of water/95% ethanol (1:1). After the appropriate interval of time -3, 4, 5 or 6 hr.- the animals were sacrificed by carbon dioxide anesthesia followed by thoractomy. The small intestine and cecum were removed and cut into segments. Contents were separated from the tissues by rinsing the segments with cold, 0.9% saline (5.0 mL). The contents were immediately diluted to 30 mL with methanol. The tissues were suspended in 0.01 $M$ $KH_2PO_4$ (5.0 mL). Then an internal standard, either prednisolone, 4, or dexamethasone, 3, depending on the experimental steroid, was added to all the samples. Both the contents and tissues were then homogenized with a Polytron Homogenizer (Brinkman Inst. Co.) at med. speed for 1-2 min. The contents were then diluted to 40 mL total volume with methanol. The tissues were diluted to 25 mL total volume with methanol. All the samples were centrifuged (5,000g, 15 min) and then the supernatant solutions were passed through membrane filters (0.45 micrometers, Versapor 800). The samples (1.4 mL) were then diluted with 0.01 $M$ $KH_2PO_4$ (0.6 mL) and 20 microliters of the resulting solution injected directly onto the HPLC column for analysis.

IN VITRO TESTING

Prednisolone glucoside, 2, (5.0 mg) or dexamethasone glucoside, 1, (5.0 mg) was incubated at 37°C in an 0.05 $M$ acetate buffer, pH 5.0 (10.0 mL) with beta-glucosidase (EC 3.2.1.21, from almonds, one unit liberates 1.0 micromole of glucose from salicin per min at pH 5.0 at 37°C). Prednisolone glucoside, 2, and dexamethasone glucoside, 1, were treated with 50 and 500 units of enzyme, respectively. At various times, aliquots (0.1 mL) were removed and quenched with methanol (9.9 mL). After centrifugation (5,000g, 10 min), the samples were diluted (1:1) with 0.01 $\underline{M}$ $KH_2PO_4$ and 20 microliters of the resulting solution injected directly onto the HPLC column for analysis.

Glucosides $\underline{1}$ and $\underline{2}$ (5.0 mg) were also incubated at 37°C with homogenized rat feces (0.5 g/10 milliliters 0.01 $\underline{M}$ phosphate buffer, pH 7.5). The feces were obtained from rats maintained on a high cellulose diet. See Shiau, S.Y., Chang, G.W. J. Nutr., 113:136 (1983). Aliquots (0.2 milliliters) were removed and quenched with MeOH (3.66 milliliters). After centrifugation (5,000g, 10 min), the samples were passed through membrane filters (0.45 micrometer pore, Versapor 450, Gelman Sciences, Inc.). They were then diluted (1:1) with 0.01 $\underline{M}$ $KH_2PO_4$. Twenty microliters of the resulting solution were injected directly onto the HPLC column for analysis.

IN VITRO METABOLISM

Rates of hydrolysis of three $\underline{p}$-nitrophenyl-glycoside substrates, $\underline{p}$-nitrophenyl-beta-D-glucoside ($\underline{p}$-NP-glc), $\underline{p}$-nitrophenyl-beta-D-galactoside ($\underline{p}$-NP-gal), and $\underline{p}$-nitrophenyl-beta-D-cellobioside ($\underline{p}$-NP-cel), were measured in homogenized contents of the rat stomach, proximal small intestine (PSI), distal small intestine (DSI) and cecum. (The entire small intestine was divided into two, equal length segments to give PSI and DSI.) Gastrointestinal contents were obtained from male, Sprague-Dawley rats (300-400 g) which had been maintained on a stock diet (Purina rat chow) and water ad libitum. The intestinal contents of each section were pooled from four animals for each assay. Pooling the gut contents from four animals would be expected to lower the SD by a factor of two. Following removal, the contents were quickly weighed, then diluted to 100 milliliters (stomach, PSI, and DSI) or 200 milliliters (cecum) with cold 0.01 $\underline{M}$ phosphate buffer, pH 7.0. The diluted contents were homogenized with a Polytron homogenizer (Brinkman Instrument Co.) at medium speed for 1-2 min and the solution pH measured. Homogenates were then stored on ice (ca 30 min). Homogenates (0.8 milliliters of stomach and PSI, 0.2 milliliters of DSI, and 0.04 milliliters of cecum) were added to the appropriate substrate solution (0.01 $\underline{M}$ phosphate buffer, pH 7.0) to give 1.0 mM substrate (total volume: 2.25 milliliters). The reaction was run at 37°C in a shaking water bath. The reaction was stopped after 10, 20, or 30 min by addition of 0.2 $\underline{N}$ NaOH (0.25 milliliters). The amount of $\underline{p}$-nitrophenol released was measured spectrophotometrically at 403 nm.

Rates of hydrolysis of glycoside prodrugs $\underline{1}$, $\underline{2}$, $\underline{5}$, $\underline{7}$, $\underline{9}$, $\underline{10}$, $\underline{11}$, $\underline{12}$, and $\underline{13}$ were measured in the same manner except that a higher concentration of each homogenate was used. Stomach and PSI contents were diluted to 50 mL with 0.01 $\underline{M}$ phosphate buffer, pH 7.0, DSI contents to 25 mL, and cecal contents to 200 mL, prior to homogenization. The homogenates (2.1 mL of stomach, PSI, and DSI and 1.7 mL of cecum) were added to the appropriate substrate solution (0.01 $\underline{M}$ phosphate buffer, pH 7.0) to give 1.0 mM substrate in a total volume of 2.5 mL. At various times up to 30 min, aliquots (0.3 mL) were removed and quenched with MeOH (4.7 mL). After centrifugation (5,000 $\underline{g}$, 10 min), the samples were diluted (1:1) with 0.01 $\underline{M}$ $KH_2PO_4$ and 20 microliters of the resulting solution was injected directly onto the HPLC column for analysis.

$K_{M(app)}$ and $V_{Max}$ Determinations. The $K_{M(app)}-V_{max}$ for the hydrolysis of $\underline{p}$-NP-glc and $\underline{p}$-NP-gal were determined using pooled cecal homogenates (200 mL) as described above. A range of substrate (56-1000 micromolar; final volume 2.25 mL) concentrations, spanning their apparent $K_M$, was used for each reaction. The amount of cecal homogenate used was 0.04 mL. Velocities were obtained, in duplicate, at 37°C in a shaking water bath and the reaction stopped by addition of 0.2 N NaOH (0.25 mL) after 15 min. Release of p-nitrophenol was measured spectrophotometrically at 403 nm. Eadie-Hofstee plots were used to determine the $K_{M(app)}$ (micromolar) and $V_{max}$ (micromolar/min/g) of both reactions. The wet weight (g), measured immediately after removal and pooling, was used throughout.

The $K_{M(app)}$ and $V_{max}$ were also measured from the hydrolysis of glycoside prodrugs 1, 2, 5, 7, 9, 10, 11 and 12. Again, cecal contents from four rats were pooled, weighed, diluted (100 mL, 0.01 M phosphate buffer, pH 7.0) and homogenized. A range of substrate concentrations (0.5-48 micromolar, final volume 2.5 mL) spanning the apparent $K_M$, was used for each reaction. The amount of cecal homogenate used was 0.8 mL. Reactions were run, in duplicate, at 37°C in a shaking water bath. After 15 min, the reactions were stopped by removing aliquots (0.3 mL) and quenching them with MeOH (4.7 mL). Following centrifugation (5,000g, 10 min), the samples were diluted (1:1) with 0.01 M $KH_2PO_4$ and 20 microliters of the resulting solution was injected directly onto the HPLC column for analysis. Again, Eadie-Hofstee plots were used to determine the $K_{M(app)}$ and $V_{Max}$.

Determination of Apparent Partition Coefficients.

The partitioning of prodrugs and free steroids between n-octanol and an aqueous phase (0.01 M phosphate buffer, pH 7.0) were determined at 37°C. Both octanol and buffer were saturated with the relevant aqueous or organic phase before use. Equal volumes (1.0 mL) of both phases were used and agitated for 30 min. The initial concentration of glycoside was 10 mM, dissolved in the aqueous phase. The intitial concentration of steroid was 10 mM dissolved in the organic phase. The amount of glycoside and free steroid in the aqueous phase at equilibrium was measured spectrophotometrically at 239 nm for the dexamethasone and fludrocortisone compounds and 242 nm for the prednisolone and hydrocortisone compounds. The concentration of glycoside or free steroid in the octanol phase was determined by difference.

Very generally, the invention discloses a colon-specific drug delivery system based on the use of a synthetic prodrug composition. The prodrug is comprised of the combination of an aglycone attached to a sugar by means of a glycosidic link. According to the invention, the aglycone is a drug composition, the sugar is a sugar recognizable as a substrate by bacterial glycosidases produced by colonic microflora, and the glycosidic link is a glycosidic link capable of being cleaved by the glycosidase enzymatic action of colonic microflora following recognition of the sugar as a substrate by the bacterial glycosidases. Also according to the invention, the prodrug combination is of sufficient size and hydrophilicity to allow it to pass through the mammalian gastrointestinal tract without being absorbed from the gaastrointestinal tract or without being significantly hydrolyzed by endogenous enzymes produced by the mammalian host. As a result, the prodrug will reach the area of the mammalian colon where the glycosidic link on the prodrug will be cleaved by the colonic bacterial glycosidases to release free drug most significantly to the colonic area of the bowel.

More specifically, the invention discloses a colon-specific drug delivery system based on the use of a synthetic prodrug composition, the prodrug being comprised of an aglycone moiety attached to a sugar moiety by means of a glycosidic link thereby producing a drug glycoside.

The aglycone moiety of the prodrug composition useful in the colon-specific drug delivery system disclosed herein will preferably be comprised of a drug that is most efficacious when its adsorption or absorption is essentially limited to the area of the colon. Such drugs include steroid and antibiotic drugs useful in the treatment of inflammatory bowel disease. When these drugs are absorbable, they will generally be more lipophilic than hydrophilic, and will be small enough to pass through the gastrointestinal mucosa, since, as is well known, lipophilic substances generally penetrate membranes more readily then hydrophilic substances do, and smaller molecules penetrate more readily than larger ones. Steroid drugs, antibiotics and cancer chemotherapeutic agents are preferred aglycone moieties for use in the prodrugs of the present invention. Especially preferred are steroid drugs, including prednisolone, dexamethasone, hydrocortisone and fludrocortisone.

The sugar moiety of the prodrug composition useful in the colon-specific drug delivery system disclosed herein will be comprised of a sugar moiety recognizable as a substrate by glycosidases produced by bacteria present in the mammalian intestine. Since the hydrolytic action of these bacterial glycosidases is the mechanism by which the glycosidic bond, linking the sugar moiety to the aglycone, is cleaved, it is essential that the glycoside and/or the sugar moiety, or portions thereof, be recognizable as a substrate by these bacterial enzymes. It is also essential that the sugar-drug combination, i.e. the drug glycoside, be of sufficient size and hydrophilicity to enable the prodrug to pass through the mammalian gastrointestinal tract without being significantly absorbed from the mammalian gastrointestinal tract or without being significantly hydrolyzed by endogenous enzymes produced by the mammalian host.

Although the principle glycosidases produced by the colonic microflora are beta-glycosidases, alpha-glycosidases are also produced. Thus both alpha and beta glycoses can be utilized as sugar moities in the synthetic drug glycosides disclosed herein. Since the beta-glycosidases are the principle colonic microfloral enzymes, beta-glycosides are preferred as prodrugs for use in the present colon-specific drug delivery system. However, those skilled in the art will recognize that certain alpha-glycosides can also be utilized.

The sugar moieties on the synthetic prodrugs will preferably be simple natural sugars. Preferred are natural monosaccharide, disaccharide and oligosaccharide hexoses and pentoses. Especially preferred are the natural monosaccharide hexoses D-glucose and D-galactose and the disaccharide D-cellobiose.

Since in the present invention the sugar moiety on the prodrug functions as a substrate for the bacterial glycosidases, those skilled in the art will recognize that modifications of these natural sugars are possible as long as the substrate specificity of the parent sugar molecules are preserved. For example, it is possible to substitute sulfur for oxygen on the sugar molecule. Such synthetic derivatives of the natural sugars are within the scope of this invention as long as the substrate specificity of the parent compound remains intact.

Those skilled in the art will also recognize that the glycone, aglycone and glycosidic linkage can probably all be varied to improve or alter the rate and location of drug release. Such variations fall within the scope of the present invention. For example, the sugar residue could be altered by modifying functional ties (See Whitaker, J.R., Principles of Enzymology for the Food Sciences, Marcel Dekker, New York, N.Y. (1972) pp. 434-442), or an oligosaccharide carrier could be used to hinder the rate of hydrolysis in vivo. Slow hydrolysis of refractory prodrugs in the colon might prove to be an effective mechanism for sustained-release. Changing the aglycone, as demonstrated herein, can also alter delivery. Furthermore, the stereochemistry of the glycosidic link might be utilized to vary rates and sites of release. A sustained release system for the small intestine based on certain alpha-glycosides could be cleaved by digestive enzymes. Absorption would then be moderated by the rate of release in the small intestine, as well as the physicochemical properties of the parent drug.

The present prodrug delivery system is based on use of synthetic drug glycoside prodrug compositions from which active drugs are liberated following hydrolysis of the prodrugs by specific glycosidases produced by intestinal bacteria. Since in man such intestinal bacteria are normally found in the area of the colon (See Draser, B.S. and Hile, M.J., "Human Intestinal Flora", Academic Press: London (1974) pp. 54-71), use of the present prodrug delivery system will usually result in liberation of active drugs in that area of the intestine. However, those skilled in the art will realize that certain disease states (e.g., regional ileitis or Crohn's disease) can also cause intestinal bacteria to accumulate in the normally sterile area of the small intestine. The accumulation occurs because, due to swelling and inflammation, ingested materials do not pass as quickly as they normally would through the small intestine. This slow-down in transit time leads to an "accumulation" of ingested material which in turn leads to an "accumulation" of bacteria that would not otherwise be found in this area of the intestine. In such cases, the present prodrug delivery system can be used to deliver active drugs to the diseased areas of the small intestine since such areas will normally coincide with the areas of the small intestine where the glycosidase producing colonic bacteria are accumulating.

It has been shown that the activity of glycosidic enzymes of the colon bacteria can be changed dramatically by diet. For example, in human subjects bean diets result in a large increase in alpha-galactosidase activity whereas oat bran diets increase beta-glucosidase activity. See Chang, G.W., Fukumoto, H. E., Gyory, C. P., Block, A. P., Kretsch, M. J. and Calloway, D. H., Fed. Proc. 38: 767 (1979) (Abstract). Since the drug delivery system of the present invention is based on substantially unique glycosidase activities of colonic microflora, and since the levels of the glycosidase enzymes of the colon bacteria can be controlled by simple dietary changes, physicians utilizing the colon-specific drug glycoside delivery system disclosed herein will have a further degree of control over their patient's treatment. For example, manipulation of glycosidase activity by diet may be very useful in standardizing glycosidase activity and also in raising enzyme activity in patients with a diseased colon where enzyme levels may be depressed.

To form a prodrug to be utilized in the present colon-specific drug delivery system, the invention teaches attachment of a sugar residue to the drug aglycone to create a synthetic drug glycoside. These drug glycosides can be synthesized by known chemical methods. See Igarashi, K., Adv. Carbohydr. Chem. Biochem. 34:243-283 (1977). Especially preferred as methods of preparing the drug glycosides disclosed herein are modifications of the Koenigs-Knorr reaction. See Meystre, C. and Miescher, K., Helv. Chim. Acta. 28:1153-1160 (1944); Koenigs, W. and Knorr, E., Ber. 34:957-981 (1901); and Igarashi, K., Adv. Carbohydr. Chem. Biochem. 34:243-283 (1977). In general, the procedure involves the treatment of a per-$\underline{O}$-acylated glycosyl halide with an alcohol in the presence of a heavy-metal salt of an organic base as the acid acceptor. As used herein, the modified methods involve coupling a per-$\underline{O}$-acylated glycosyl halide with an appropriate steroid in chloroform in the presence of silver carbonate as acid acceptor. See FIGURE 1. The acetyl protecting groups on the sugar residues of the drug glycosides are then removed by treatment with bases to yield synthetic drug glycosides.

Use of the modified Koenigs-Knorr reaction to prepare drug glycosides is illustrated herein with the preparation of glucoside 2 (and its acetyl precursor PREDTAGLU) and glucoside 1 (and its acetyl precursor DEXATAGLU). See Example I. As discussed further in Example II, the modified Koenigs-Knorr reaction has also been used to produce a variety of other drug glycosides useful in the drug delivery system of the present invention. These drug glycosides include: prednisolone-21-beta-D-galactoside, 10; dexamethasone-21-beta-D-galactoside, 9; hydrocortisone-21-beta-D-glucoside, 5; hydrocortisone-21-beta-D-galactoside, 11; fludrocortisone-21-beta-D-glucoside, 7;

fludrocortisone-21-beta-D-galactoside, 12. Example II further illustrates the preparations and use of drug glycosides consisting of a disaccharide coupled to a drug. Such drug glycosides are represented by prednisolone-21-beta-D-cellobioside, 13.

In utilizing the synthetic drug glycosides as prodrugs in the present colon-specific drug delivery system, the prodrug is administered orally or intragastrically to the mammalian host. The prodrug is then allowed to pass through the mammalian host's gastrointestinal system. Since the synthetic prodrug is larger and more hydrophilic than the parent drug, the prodrug is less permeable than the parent drug. In addition, since the glycosidic bond linking the glycose to the aglycone is a bond that will be substantially selectively cleaved by glycosidic bacterial enzymes produced by colonic microflora, the synthetic prodrug will pass through the gastrointestinal tract without being significantly absorbed from the gastrointestinal tract or without being significantly hydrolyzed by endogenous enzymes produced by the mammalian host. Once in the area of the colon, the prodrug will be acted upon by bacterial glycosidases, thus releasing free drug for adsorption to or absorption by the colonic mucosa.

In a preferred form of the present invention the synthetic prodrugs are comprised of the 21-yl beta-D-glucosides and galactosides of dexamethasone, prednisolone, hydrocortisone, and fludrocortisone, and the 21-yl beta-D-cellobioside of prednisolone. Especially preferred is the steroid glycoside dexamethasone-21-beta-D-glucoside (1).

Synthesis of dexamethasone-21-beta-D glucoside, 1, and prednisolone-21-beta-D-glucoside, 2, using a modified Koenigs-Knorr reaction, is shown in Example I. In preparing these synthetic drug glycosides, the bromosugar, 2,3,4,6-tetra-_O_-acetyl-l-bromo-alpha-D-glucopyranose, was coupled with the appropriate steroid in chloroform in the presence of silver carbonate as acid acceptor. Acetyl glycoside products were isolated and then the acetyl protecting groups on the sugar residues of the glycosides removed by treatment with base.

The two synthetic drug glycosides were then used in the colon-specific drug delivery system disclosed herein. As shown more specifically in Example I, drug glycosides 1 and 2 were administered intragastrically to mammalian rat hosts. Analysis of the hosts' intestinal contents and tissues revealed that in this host, both drug glycosides 1 and 2 reached the colon within 4-5 hours, where they were rapidly hydrolyzed. Although delivery of dexamethasone (via glycoside 1) appeared to be more specific than that of prednisolone (via glycoside 2) in the rat host, both steroid drugs reached the area of the colon when administered via the colon-specific drug delivery system disclosed herein. (Nearly 60% of an oral does of glycoside 1 reached the cecum whereas less than 15% of glucoside 2 did.) In contrast, when the free drug steroids prednisolone and dexamethasone were administered orally, they were absorbed almost exclusively from the small intestine.

The influence of prodrug structure on specificity of glycoside/glycosidase based colon-specific drug delivery was studied by preparing seven additional steroid glycosides (See Example II), measuring their relative lipophilicities (See Example V) and hydrolyzing them with bacterial glycosidases from rat intestines (See Examples III and IV). Preparation of compounds 1 and 2 is outlined in Example I. Preparation of the seven additional compounds is outlined in Example II. (The nine steroid glycosides are comprised of the 21-yl beta-D-glucosides and galactosides of dexamethasone, prednisolone, hydrocortisone and fludrocortisone, and the 21-yl beta-D-cellobioside of prednisolone.)

As illustrated in Examples III and IV, the nine deacetylated glycoside prodrugs, along with the p-nitrophenyl derivatives of beta-D-glucoside, galactoside, and cellobioside, were subjected to hydrolysis by the contents of the rat stomach, proximal small intestine (PSI), distal small intestine (DSI), and cecum. As the data in Example III demonstrate, all the prodrugs were hydrolyzed slowly by PSI and stomach contents, more rapidly by contents of the DSI, and most rapidly by cecal contents. However, the prodrugs themselves had very different susceptibilities to hydrolysis. Hydrolysis rates catalyzed by DSI contents decreased in the following order: prednisolone 21-yl beta-D-galactoside (10) > prednisolone 21-yl beta-D-glucoside (2) > prednisolone-21-yl beta-D-cellobioside (13) > dexamethasone 21-yl beta-D-galactoside (9) > dexamethasone 21-yl beta-D-glucoside (1). Hydrolysis of cellobioside 13 was only half that of glucoside 2 and one-fourth that of galactoside 10. Hydrolysis of all the prodrugs in cecal contents was rapid, with the exceptions of hydrocortisone 21-yl beta-D-glucoside (5) and fludrocortisone 21-yl beta-D-glucoside (7), which were hydrolyzed more slowly than the other glucoside prodrugs. Eadie-Hofstee plots were used to determine the $K_{M(app)}$ and $V_{MAX}$ of the reactions. The Eadie-Hofstee plots suggested that bacterial beta-D-glucosidase activity in the colon may be more heterogeneous in nature than beta-D-galactosidase activity. See Example IV.

Since the physicochemical properties of the prodrug can influence the specificity of the present delivery system and since lipophilicity is very important in determining rates of penetration across biological membranes, gastrointestinal absorption as predicted by octanol-buffer partition coefficients was also investigated. More specifically, the relative lipophilicities of the prodrugs and free steroids were compared by measuring their octanol-buffer partition coefficients (P). Log P of the free steroids ranged from 1.54 to 1.73. Log P of the prodrugs ranged from 0.11 to 0.84, except for the logarithm of the P of cellobioside 13 which was considerably lower (-0.56). See Example IV.

Those skilled in the art will realize that the specificity of delivery of a glycoside prodrug to the lower intestine can be estimated from its rate of hydrolysis by DSI contents and its octanol-buffer partition coefficient since these parameters limit the amount of prodrug which can survive premature hydrolysis or absorption in the DSI. For example, under the assay conditions used in this study, glucoside 1 supported a specific activity of hydrolysis of 19 nmole/min/g in DSI contents and had a log P value of 0.59. When this prodrug was administered orally to rats, about half of the dose reached the cecum. Even more effective delivery could be expected of any prodrug which supports less hydrolysis by DSI contents and which has a lower log P value.

The data from the in vivo and in vitro tests indicate that use of a disaccharide such as cellobiose, or even a trisaccharide, as the hydrophilic moiety, can produce a prodrug which will be superior in some instances. Such oligosaccharide carriers might be essential if smaller or more lipophilic drugs are to be delivered to the large intestine. In addition, the in vitro·data from Examples II-IV indicate that the relative rates of hydrolysis, and the relative lipophilicities can be used in conjunction with the in vivo data from Example I to enable one skilled in the art to estimate the site-specificity of glycoside prodrugs prior to extensive animal studies.

Thus it can be seen that the drug glycosides of the present invention have the property of undergoing bacterial cleavage of their glycosidic bond under conditions found in the gastrointestinal tract of mammals. The free drug liberated by such cleavage is absorbable through the colonic mucosa. Based on the art's teaching of the pharmacological efficacy of the free drugs, administration of the drug via the prodrug is thus shown to be possible.

Specific embodiments of the present invention are outlined in the following examples. Such examples are for illustrative purposes only and are not intended to limit the scope of the claims in any way.

EXAMPLE I

The teaching of the present invention makes it possible to construct a variety of synthetic drug glycosides useful in the disclosed colon-specific drug delivery system. This example compares the use of two such steroid glycosides, dexamethasone-21-beta-D-glucoside, (1), and prednisolone-21-beta-D-glucoside, (2), in the disclosed colon-specific delivery system.

Dexamethasone and prednisolone are steroid drugs useful in the treatment of inflammatory bowel disease. Prodrugs 1 and 2 are the respective drug glycosides of these two steroid compounds. To test the efficacy of 1 and 2 in the colon-specific drug delivery system disclosed herein, the prodrugs were administered intragastrically to rats. The intestinal contents and tissues were then analyzed to determine where the steroids were released and absorbed. Prednisolone and dexamethasone were also administered to compare the absorption of the drug glucosides with the free steroids. Both drug glucosides 1 and 2 were found to reach the colon in 4-5 hr., where they were rapidly hydrolyzed. Delivery of dexamethasone (via glucoside 1) to the rat lower intestine appeared to be more specific than that of prednisolone (via glucoside 2): 60% of an oral dose of glucoside 1 and 15% of an oral dose of glucoside 2 reached the cecum. In contrast, when the free steroids prednisolone and dexamethasone were administered orally, they were absorbed almost exclusively from the small intestine: less than 1% of the oral doses reached the cecum.

Glucosides 1 and 2 were prepared by glycosylation of dexamethasone and prednisolone using a modified Koenigs-Knorr reaction. See Meystre, C. and Miescher, K., Helv. Chim. Acta. 28:1153-1160 (1944); and Koenigs, W. and Knorr, E., Ber. 34:957-981 (1901). The bromosugar, 2, 3, 4, 6-tetra-O-acetyl-1-bromo-alpha-D-glucopyranose, was coupled with the appropriate steroid in carbon tetrachloride in the presence of silver carbonate as acid acceptor. See FIGURE 1. Acetyl glycoside products were isolated from the reaction mixture by column chromatography on reversed-phase (C-18) packing material. Yields were 38% for PREDTAGLU, 15, and 25% for DEXATAGLU, 14. These fair yields are typical for this reaction. See Igarashi, K., Adv. Carbohydr. Chem. Biochem. 34:243-283 (1977).

Proton NMR confirmed that the glucosides formed were beta linked. The anomeric proton (C-1') exhibited a doublet at 4.20 ppm for PREDTAGLU, 15, and 4.18 ppm for DEXATAGLU, 14. The coupling constants were 8 Hz for both compounds. These resonance signals indicate a trans-diaxial relationship between the vicinal C-1', 2' protons. See Williamson, D.G., Collins, D.G., Layne, D.S. and Bernstein, S., Biochemistry 8:4299-4304 (1969).

The acetyl protecting groups on the sugar residues of PREDTAGLU, 15, and DEXATAGLU, 14, were removed by treatment with 0.01 N sodium hydroxide. The $^1$H NMR of these compounds again provided evidence of the stereochemistry at their anomeric carbons (beta-linked). Also, treatment of both glucoside 2 and glucoside 1 with commercial beta-glucosidase led to the removal of the glucose moiety in each case. Glucoside 2 was hydrolyzed several orders of magnitude faster than was glucoside 1. In addition, incubating these glucosides with homogenized rat feces resulted in extensive hydrolysis of each.

Separation of the glycosides from the free steroids was performed by HPLC. A typical chromatogram of the cecal contents of a rat given glucoside 1 intragastrically and sacrificed 6 hr. later is shown in FIGURE 2. Peak A is glucoside 1; Peak B is dexamethasone, 3; and Peak C is prednisolone, 4, which was added prior to homogenization as an internal standard. (The Altex 5 micrometer Ultrasphere, C-18 column was eluted with MeOH/0.01 M KH$_2$PO$_4$ (56.5:43:5) at a flow rate of 1.2 mL/min.)

The recovery of glucoside 1 and free steroid 3 from the small intestine and cecum at various times following oral administration of glucoside 1 is given in Table I, supra. As the data in the table illustrate, after 3 and 4 h, glucoside 1 was recovered primarily in the lower small intestine. By 5 h, very little of glucoside 1 was observed in either the small intestine or cecum. (At the same time, large amounts of steroid 3 were recovered from the cecum.) However, as glucoside 1 passed from the lower small intestine into the cecum, the free drug was released rapidly. The fact that some free steroid was detected in the small intestine at the times tested indicated that some hydrolysis occurred before the prodrug reached the cecum or colon.

The recovery of steroid glucosides and free steroids from the intestinal contents and tissues is also shown graphically in FIGURE 3. In that figure, recovery of glucoside 1, (DEXAGLU), and dexamethasone 3, (DEXA), from rats given 7.5 mg of glucoside 1 at 0 hr are shown in panels A and B. Recovery of glucoside 2 (PREDGLU) and prednisolone 4 (PRED) from animals given 7.5 mg of glucoside 2 at 0 hr are shown in panels C and D. Data are given as means ±SEM (n=4). Solid circle and triangle symbols indicate intestinal contents; open circle and triangle symbols indicate intestinal tissues.

The results reveal that overall, the delivery of glucoside 1 and subsequent release of steroid 3 in the rat cecum was quite specific. At 4 h, 59% of the administered dose of glucoside 1 was recovered from the lower small intestine contents unhydrolyzed. If all this glucoside passed into the cecum, then nearly 60% of the dose would have been delivered specifically to the cecum. At 5 h, an average of 2.24 mg (or an equivalent of 44% of the administered dose) was recovered in the cecum as free steroid. The difference (59% vs. 44%) was probably due to absorption of free drug by the cecal mucosa following hydrolysis of the prodrug in the cecum.

The recovery of glucoside 2 and free steroid 4 from the small intestine and cecum at various times following oral administration of glucoside 2 is given in Table II supra. Recovery of glucoside 2 after 3 and 4 h from the small intestine was much lower than that for glucoside 1. By 5 h, some free steroid was found in the cecum, however the specificity was quite low. Free steroid 4 was detected in the small intestine at all time points tested, again indicating the presence of glycosidases in the rat small intestine. The recovery of glucoside 2 and steroid 4 from the intestinal contents and tissues is shown graphically in panels C and D of FIGURE 2.

The results indicate that delivery of glucoside 2 was less efficient than that of glucoside 1. Only 14.8% of the administered dose of glucoside 2 could be recovered as such from the lower small intestine after 4 h. Therefore, only about 15% of the dose could have been delivered to the cecum. By 5 h, an average of 0.57 mg of steroid 4 (or an equivalent of 11% of the administered dose) was recovered in the cecum. Again, the difference (14.8% vs. 11%) is probably due to absorption of steriod 4 into the systemic circulation following hydrolysis in the cecum.

No glucoside or free steroid was recovered from the colon of those animals tested. This was probably due to slow transit times and the fact that the time points tested following administration did not allow for any released steroid to pass into the rat colon. Transit times in this experiment were slower, but still close to the value of 6.6 ± 0.4 h reported for passage through the alimentary canal of rats fed a stock diet. See Williams, V.J., Senior, W., Aust. J. Biol. Sci., 35:373 (1982).

The specificity of drug release was evaluated further by comparing the difference in free steroid recovered in the small intestine and in the cecum. A paired t-test indicated that the preferential release of free steroid 3 in the cecum over that in the small intestine was statistically significant (t = 2.32, p < 0.025). A similar analysis of recoveries of steroid 4 showed that the preferential release of 4 in the cecum was not quite statistically significant (t = 1.72, 0.05 < p < 0.10).

Intestinal transit times varied greatly, and in many cases the administered dose did not reach the cecum by the time of sacrifice. When these animals were excluded from the statistical calculations, the specificity of release is greater for both steroids 2 and 4. Measurable amounts of glucoside 1 reached the cecum in eleven of the sixteen animals tested. Analysis of data from only these eleven animals showed that the preferential release of steroid 3 had high statistical significance (t = 3.17, p < 0.01). For glucoside 2, it was found that the administered dose reached the cecum in eight of sixteen animals tested. Data from these eight animals indicated that the preferential release of steroid 4 was statistically significant (t = 3.94, p < 0.005). However, the combined total recoveries of glucoside 2 and steroid 4 at 4 and 5 h was very low. Therefore the efficiency of drug delivery to the cecum was very low, despite the calculated significance.

Control experiments in which unmodified steroids 3 and 4 were administered showed that they were absorbed almost completely from the small intestine. These data are shown in FIGURE 4. In that figure, recovery of steroid from animals given 5.25 mg of dexamethasone (DEXA), 3, is shown in panel A; recovery of steroid from animals given 5.10 mg of prednisolone (PRED), 4, is shown in panel B. Data points are means ± SEM (n=3) from intestinal contents, shown as solid circles, and tissues, shown as open circles.

The system illustrated in this example is based on the release of anti-inflammatory steroids from poorly absorbed steroid glycosides in the rat cecum. Despite the obvious anatomical differences between the laboratory rat and man, the rat cecum can be considered to be a satisfactory model for the proximal colon of man. Both organs are recipients of digesta from the small intestine and both are sites of large bacterial populations and extensive microbial activity. Therefore, the term "lower intestine" is used herein for either the combined rat cecum and colon or the human colon with its poorly defined cecal area.

While the rat model is useful, it suffers from the problem of a relatively high bacterial population and a subsequently high level of glycosidase activity that is present in the stomach, upper small intestine, and lower small intestine. For example, there are an average of $10^{7.7}$, $10^{6.9}$, and $10^{7.7}$ microorganisms/g wet weight in the rat stomach, upper small intestine, and lower small intestine, respectively. In contrast, the bacterial population in man's stomach and small intestine is much lower. There are only an average of $10^{0}$, $10^{2.6}$, and $10^{4.2}$ microorganisms/g wet weight residing in the human stomach, upper small intestine, and lower small intestine, respectively. See Drasar, B.S. and Hill, M.J., "Human Intestinal Flora", Academic Press: London (1974) pp. 54-71, and Hawksworth, G., Drasar, B.S. and Hill, M.J., J. Med. Microbiol. 4:451 (1971). Bacteroides and Bifidobacteria are the bacterial species comprising the majority of microorganisms in the gastrointestinal system of both the laboratory rat and man. In addition, both species have been shown to produce measurable quantities of beta-glucosidase *in vivo*. *See* Hawksworth et al., *supra*.

Despite the high level of microbial activity in the rat upper intestine, glucoside 1 showed remarkable specificity towards the lower intestine. This prodrug should be even more specific when used in man because the microbial activity in human stomach and small intestine is much lower than that of the rat. Sulfasalazine, a prodrug used successfully in man, requires activation by colonic microflora. *See* Peppercorn, M.A. and Goldman, P., *J. Pharm. Exp. Ther.* 181:55 (1972), and Peppercorn, M.A. and Goldman, P., *Gastroenterology* 64:240 (1973). Sulfasalazine works on much the same principle as does the present glycoside/glycosidase delivery system. Therefore, based on the similarities between these two prodrug delivery systems, and the degree of specificity of glucoside 1 demonstrated in the rat model, it is likely that certain drugs can be effectively delivered to the colon of man via glycoside prodrugs.

It is worth noting that the relatively poor performance of glucoside 2 in the rat model could be due to several factors. Although it is possible that glucoside 2 was absorbed more extensively from the stomach and small intestine than was glucoside 1, it was more likely that glucoside 2 was hydrolyzed to a greater extent in the stomach and small intestine than was glucoside 1. Commercial beta-glucosidase was much more active towards glucoside 2 than towards glucoside 1. Similar factors may be functioning in the rat gastrointestinal tract.

<u>EXAMPLE II</u>

This example illustrates synthesis of seven additional drug glycosides useful in the colon-specific drug delivery system of the present invention.

Using the modification of the Koenigs-Knorr reaction discussed *supra*, a number of additional drug glycosides have been synthesized. Some of these drug glycosides are listed below. The compounds in parentheses are the protected glycoside products produced in the first step of the modified synthesis. *See* FIGURE 1. The protecting groups (acetyl functions) were removed with base to give the glycosides preceding the compounds in parentheses. The compounds include: prednisolone-21-beta-D-galactoside, 10, (and pred.-21-yl 2,3,4,6-tetra-O-aceto-beta-D-galactoside, 19); dexamethasone-21-beta-D-galactoside, 9, (and dexa.-21-yl 2,3,4,6-tetra-O-acetyl-beta-D-galactoside, 18); hydrocortisone-21-beta-D-glucoside, 5, (and hydrocort.-21-yl 2,3,4,6-O-tetra-O-acetyl-beta-D-glucoside, 16); hydrocortisone-21-beta-D-galactoside, 11, (and hydrocort.-21-yl 2,3,4,6-tetra-O-acetyl-beta-D-galactoside, 20); fludrocortisone-21-beta-D-glucoside, 7, (and fludrocort.-21-yl 2,3,4,6-tetra-O-acetyl-beta-D-glucoside, 17); fludrocortisone-21-beta-D-galactoside, 12, (and fludro-21-yl 2,3,4,6-tetra-O-acetyl-beta-D-galactoside, 21), and prednisolone-21-beta-D-cellobioside, 13, (and prednisolone-21-yl heptaacetyl-beta-D-cellobioside, 22).

Proton NMR spectroscopy of the acetyl glycosides indicated a beta-linkage at their anomeric carbons. All the compounds exhibited a doublet at approximately 4.2 ppm for the anomeric proton with coupling constants ranging from 7.2 to 8.0 Hz. These resonance signals indicate a *trans*-diaxial relationship between the C-1',2' protons. *See* Williamson, D.G., Collins, D.C., Layne, D.S., Conrow, R.B., and Bernstein, S., *Biochemistry* 8:4299 (1969).

Removal of the acetyl protecting groups on the sugar residues was accomplished by base catalyzed hydrolysis using 0.01 $\underline{N}$ NaOH in MeOH. Yields ranged from 60 to 83% for this step. $^1$H NMR spectroscopy confirmed that the free glycosides were still beta-linked.

### EXAMPLE III

Results from the study of the present glycoside/glycosidase based delivery system in the laboratory rat indicate that certain anti-inflammatory steroids can be delivered to the lower intestine with varying degrees of specificity, depending on the aglycone. To better understand the factors controlling specificity, and to demonstrate the behavior of additional drug glycosides in the mammalian gastrointestinal tract, the kinetics of release of aglycones from three $\underline{p}$-nitrophenyl-glycosides and nine steroid glycoside prodrugs were measured using freshly prepared homogenates from contents of the rat stomach, proximal small intestine (PSI), distal small intestine (DSI), and cecum.

More specifically, total activities (micromoles/min) and specific activities (nmoles/min/g) of native enzymes were first measured in stomach, PSI, DSI, and cecal content homogenates using three $\underline{p}$-nitrophenyl-glycoside substrates. This was accomplished by following the release of $\underline{p}$-nitrophenyl from $\underline{p}$-nitrophenyl-beta-D-glucoside ($\underline{p}$-NP-glc) via beta-D-glucosidase, $\underline{p}$-nitrophenyl-beta-D-galactoside ($\underline{p}$-NP-gal) via beta-D-galactosidase, and $\underline{p}$-nitrophenyl-beta-D-cellobioside ($\underline{p}$-NP-cel) via beta-D-cellobiosidase and beta-D-glucosidase. Results of these measurements are shown in Table III. Release of $\underline{p}$-nitrophenol from all three substrates in all four segments tested indicated the presence of glycosidase activity all along the rat gastrointestinal tract.

Total activities of each glycosidase were generally lowest in the stomach and PSI, higher in the DSI, and highest in the cecum. Specific activities of the three glycosidases followed the same general pattern, with highest activities found in the cecum. In addition to the gradient of glycosidase activity along the gastrointestinal tract, there were differences in enzyme levels. Specific activity of cecal beta-D-galactosidase was about four times that of cecal beta-D-glucosidase, and about sixteen times that of cecal beta-D-cellobiosidase.

Total activities and specific activities for hydrolysis of all nine glycoside prodrugs disclosed in Examples I and II were also measured. Results of those measurements are given in Tables IV (glucosides 1, 2, 5, 7) and V (galactosides 9, 10, 11, 12, and cellobioside 13). The tables are shown supra. Total and specific activities for the hydrolysis of all prodrugs were relatively low in the stomach and PSI contents. They increased in the DSI and were highest in the cecal content homogenates. This pattern follows that found for the hydrolysis of the p-NP-glycosides; however, in all homogenates tested, total and specific activities for hydrolysis of each type of prodrug (glucoside, galactoside, and cellobioside) were considerably lower than were those of the corresponding p-NP-glycosides.

The data indicate that hydrolysis of all prodrugs was relatively slow when incubated with homogenates of contents from the stomach and PSI, faster in the DSI homogenates, and fastest in the cecum. Loss of prodrugs through hydrolysis in the stomach and PSI is probably negligible. Transit through this portion of the rat gastrointestinal tract is quite rapid: transit times can be as low as 40 min.

This combination of low enzyme activity and rapid transit in the rat stomach and PSI means that specificity of delivery is probably a function of glycosidase activity and residence time in the DSI. Transit slows considerably in the DSI, increasing the possibility of premature hydrolysis and less specific drug-delivery to the rat cecum.

Differences in total and specific activities became apparent when each prodrug was incubated with the homogenates of the DSI contents. Dexamethasone prodrugs 1 and 2 were more resistant to hydrolysis than the other prodrugs. Of the two glucosides tested in vivo, glucoside 1 was found to be hydrolyzed about three times slower in the homogenates of the DSI contents than was glucoside 2. As shown in the animal tests included in Example I, glucoside 1 was delivered to the rat lower intestine about four times more specifically than was glucoside 2 (59% for 1 and 14% for 2). Therefore, there is a rough correlation between total and specific activities in homogenates of DSI contents, and specificity of delivery observed in vivo. Because the other prodrugs were all hydrolyzed to much greater extent in DSI homogenates, they may not be delivered to the lower intestine as specifically as glucoside 1.

All the prodrugs except glucosides 5 and 7 were hydrolyzed much faster by cecal contents than by DSI contents. The galactosides were hydrolyzed more rapidly by cecal homogenates than were their glucoside counterparts. Thus, any prodrug reaching the cecum following oral administration would be expected to rapidly liberate the pharmacon as desired, with the possible exception of glucosides 5 and 7. However, the galactoside prodrugs would probably release significant amounts of their free steroids in the DSI, prior to

-50-

reaching the lower intestine. Therefore, the galactoside prodrugs described herein would probably be poor candidates for drug delivery to the rat cecum. It should be noted that the human intestine has a much sharper gradient of bacterial colonization; compounds which are not delivered specifically in the rat may be in the human intestine. Furthermore, there may be applications in which it is desirable to deliver a drug to the DSI.

When compounds 13, 10 and 2 were incubated in DSI or cecal homogenates, the rate of release of prednisolone (4) from prednisolone-cellobioside (13) was much slower than that of steroid 4 from either glucoside 2 or galactoside 10. As a result, delivery of steroid 4, via cellobioside 13, to the rat cecum would probably still not be as specific as that of dexamethasone (3), via prodrug 1 or 9. However, the prednisolone-glycosides were the most enzymatically labile prodrugs investigated. A cellobioside derivative of any of the other three steroids would probably improve their specificity of delivery. Release of steroid 4 from cellobioside 13 in the rat cecum would probably be rapid, approximately the same as was observed in vivo for release of steroid 3 from glucoside 1. Slow release of steroid 4 from cellobioside 13 is probably due to the fact that the rate of hydrolysis by beta-D-cellobiosidase is slower than the two step hydrolysis by beta-D-glucosidase.

### EXAMPLE IV

$K_M$(app) and $V_{Max}$ Determinations.

The $K_M$-$V_{Max}$ data for hydrolysis of the p-nitrophenyl-beta-D-glucoside (p-NP-glc), p-nitrophenyl-beta-D-galactoside (p-HP-gal), and the glucoside and galactoside prodrugs are given in Table VI.

-51-

Eadie-Hofstee plots were used to determine the $K_{M(app)}$ and $V_{MAX}$ of the enzyme reactions. The Eadie-Hofstee plots (of the relationship V vs V/[S]) for hydrolysis of p-NP-glc and p-NP-gal in homogenates of pooled cecal contents from four animals are presented in Figures 5 and 6. At lower substrate concentrations, the plot of the velocity-substrate relationship for hydrolysis of p-NP-glc deviated from linearity. Hydrolysis of p-NP-gal was, however, essentially linear.

In calculating the $K_M$ and $V_{Max}$ values for hydrolysis of p-NP-glc, p-NP-gal, and the glucoside and galactoside prodrugs, it was found that the beta-D-glucosidase activity may be more heterogeneous in nature than beta-D-galactosidase activity. This was seen in the Eadie-Hofstee plots, as shown in Figures 5 and 6, and may reflect the production of different beta-D-glucosidases by the many bacterial species living in the large intestine.

## EXAMPLE V

Partition coefficients, often measured between n-octanol and an aqueous phase, have proved very useful in correlating lipophilicity with absorption patterns of compounds absorbed by passive diffusion from the gastrointestinal tract. See Leo, A., Hansch, C., and Elkins, D., Chem. Rev. 71:525 (1971). Therefore, partition coefficients of the prodrugs and the free steroids were measured.

More specifically, the apparent n-octanol-buffer (0.01 M phosphate buffer, pH 7.0) partition coefficients (P) were measured at 37°C. The results of these measurements, expressed as log P, are given in Table VII. Cellobioside 13 had the lowest log P (-0.56) of any of the prodrugs tested. The galactoside prodrugs all had lower log P values than the corresponding glucoside prodrugs. As expected, the log P values for the free steroids were much greater than those of the glycosides.

The effect of incorporating a hydrophilic moiety (i.e., glucose) into dexamethasone is shown in Example I. More specifically, the data contained therein show that following oral administration of a glucose/dexamethasone drug glycoside (glucoside 1), 78% of the dose was recovered intact from the animals' intestinal lumen 3 h later; in contrast, only 3.9% of an oral dose of steroid 3 was recovered 3 h later. Thus, attaching a hydrophilic glucose moiety to steroid 3 drastically impeded its absorption. This is reflected in the difference in partition coefficients (log P) of glucoside 1 (0.59) and steroid 3 (1.72). Because the log P of glucoside 2 was even lower than that of glucoside 1, it appears that the poorer specificity of delivery of glucoside 2 in the rat model was due primarily to premature release of the drug in the upper intestine rather than absorption from the gastrointestinal lumen prior to reaching the cecum.

TABLES

The following tables are referred to infra:

Table I.  Recovery of glucoside 1 and steroid 3 from
the small intestine and cecum at various
times after administration of 7.5 mg of
glucoside 1.

| time (h) | small intestine | | cecum | |
|---|---|---|---|---|
| | 1 | 3 | 1 | 3 |
| | (glucoside) | (steroid) | (glucoside) | (steroid) |
| 3 | 5.61 mg[a] | 0.13 mg | 0.21 mg | 0.09 mg |
| 4 | 5.00 | 0.11 | 0.02 | 0.05 |
| 5 | 0.24 | 0.12 | 0.23 | 2.24 |
| 6 | 0.94 | 0.18 | 0.04 | 1.66 |

[a] values represent the average of 4 animals.

Table II. Recovery of glucoside 2 and free steroid 4
from the small intestine and cecum at various
times after administration of 7.5 mg of
glucoside 2.

| time | small intestine | | cecum | |
|---|---|---|---|---|
| (h) | 2 | 4 | 2 | 4 |
| | (glucoside) | (free steroid) | (glucoside) | (free steroid) |
| 3 | 1.57 mg[a] | 0.30 mg | 0.0 mg | 0.0 mg |
| 4 | 1.73 | 0.18 | 0.0 | 0.0 |
| 5 | 0.19 | 0.09 | 0.0 | 0.57 |
| 6 | 0.18 | 0.03 | 0.06 | 0.29 |

[a] values represent the average of 4 animals.

Table III.    Hydrolysis of p-nitro phenyl-glucoside, p-nitro phenyl-galactoside, and p-nitro phenyl-cellobioside by the Contents of Different Segments of Rat Intestine.[a]

| Intestinal segment | Substrate | | | | | |
|---|---|---|---|---|---|---|
| | p-NP-glc | | p-NP-gal | | p-NP-cel | |
| | Tot. act. | Sp. act. | Tot. act. | Sp. act. | Tot. act. | Sp. act. |
| stomach[b] | 0.08 | 72.2 | 0.70 | 57.8 | 0.35 | 34.2 |
| PSI[c] | 0.15 | 43.2 | 0.20 | 58.3 | 0.05 | 7.2 |
| DSI | 0.65 | 86.6 | 3.6 | 425 | 0.25 | 26.0 |
| cecum | 9.1 | 454 | 30.8 | 1620 | 2.6 | 96.5 |

[a] Tot. act.: activity of contents of the entire intestinal segment, expressed as micromole/min. Sp. act.: specific activity of contents of the intestinal segment, expressed as nmoles/min/g wet weight.

[b] pH of stomach homogenates was ca 5.0.

[c] pH of PSI, DSI, and cecal homogenates was 7.0.

Table IV. Hydrolysis of Glucoside Prodrugs 1, 2, 5, and 7 by the Contents of Different Segments of Rat Intestine.[a]

| Intestinal segment | Prodrug | | | | | | | |
| --- | --- | --- | --- | --- | --- | --- | --- | --- |
| | 1 | | 2 | | 5 | | 7 | |
| | Tot. act. | Sp. act. | Tot. act. | Sp. act. | Tot. act. | Sp. act. | Tot. act. | Sp. act. |
| stomach | 0.12 | 6.8 | 0.12 | 6.8 | 0.09 | 5.5 | 0.14 | 7.7 |
| PSI | 0.07 | 11.0 | 0.07 | 12.2 | 0.10 | 15.9 | 0.08 | 12.3 |
| DSI | 0.34 | 19.3 | 1.0 | 56.8 | 0.70 | 39.2 | 0.43 | 33.0 |
| cecum | 3.5 | 141 | 6.6 | 265 | 0.76 | 30.5 | 1.4 | 42.0 |

[a] Measured by following the release of the free steroid by HPLC. Abbreviations and units are the same as those in Table II.

Table V.  Hydrolysis of Galactoside Prodrugs 9, 10, 11, 12, and
Cellobioside Prodrug 13 in Contents of Different
Segments of Rat Intestine.[a]

| Intestinal | Prodrug | | | | | | | | | |
|---|---|---|---|---|---|---|---|---|---|---|
| segment | 9 | | 10 | | 11 | | 12 | | 13[b] | |
| | Tot. act. | Sp. act. | Tot. act. | Sp. act. | Tot. act. | Sp. act. | Tot. act. | Sp. act. | Tot. act. | Sp. act. |
| stomach | 0.004 | 2.0 | 0.19 | 12.1 | 0.02 | 12.5 | 0.01 | 7.0 | 0.01 | 7.5 |
| PSI | 0.004 | 11.7 | 0.23 | 49.3 | 0.02 | 42.5 | 0.01 | 12.5 | 0.11 | 26.0 |
| DSI | 0.41 | 29.3 | 1.72 | 122 | 1.6 | 110 | 0.80 | 56.7 | 0.64 | 32.5 |
| cecum | 6.5 | 322 | 13 | 665 | 11.9 | 592 | 12.9 | 642 | 2.2 | 109 |

[a] Measured by following the release of the free steroid by HPLC.
Abbreviations and units are the same as in Table I.

[b] Expressed as release of free steriod 4.

Table VI.  $K_{m(app)}$ (micromolar) and $V_{max}$ (micromolar/min/g) for

Hydrolysis of p-NP-glc, p-NP-gal, and

Prodrugs 1, 2, 5, 7, 9, 10, 11, and 12.[a]

| substrate | $K_{m(app)}$ | $V_{max}$ |
|---|---|---|
| p-NP-glc[b] | 360 | 697 |
| p-NP-gal[b] | 166 | 1233 |
| glucoside 1[c] | 2.5 | 2.2 |
| glucoside 2[c] | 4.5 | 2.6 |
| glucoside 5 | 5.7 | 2.6 |
| glucoside 7 | 4.4 | 2.4 |
| galactoside 9 | 13.3 | 3.9 |
| galactoside 10 | 20.0 | 10.3 |
| galactoside 11 | 16.7 | 10.2 |
| galactoside 12 | 33.3 | 20.6 |

[a] Measurements made in pooled cecal content homogenates.

[b] Reaction followed by measuring the release of p-nitrophenol spectrophotometrically at 403 nm.

[c] Measured by following the release of free steroid using HPLC.

Table VII.  Apparent $\underline{n}$-Octanol-Buffer Partition
Coefficients (Log P).[a]

| compd | Log P |
|---|---|
| glucoside 1 | 0.59 |
| glucoside 2 | 0.27 |
| glucoside 5 | 0.44 |
| glucoside 7 | 0.84 |
| galactoside 9 | 0.49 |
| galactoside 10 | 0.11 |
| galactoside 11 | 0.15 |
| galactoside 12 | 0.25 |
| cellobioside 13 | −0.56 |
| steroid 3[b] | 1.72 |
| steroid 4[c] | 1.55 |
| steroid 6[d] | 1.54 |
| steroid 8[e] | 1.73 |

[a] Agitated for 30 min. at 37°C with concentration determined in the aqueous phase.

[b] Lit. (Leo, A., Hansch, C. and Elkins, D., Chem. Rev. 71:525 (1971)) values:  1.90, 1.59 (both using diethyl ether as organic phase).

[c] Lit. (Leo, et al., supra, value:  1.42.

[d] Lit. (Leo, et al., supra, values:  1.53, 0.96 (diethyl ether), 0.89 (benzene), 1.93 (iso-butyl alcohol).

[e] Lit. (Leo, et al., supra, value:  1.68.

The foregoing description and accompanying examples demonstrate the efficacy of a colon-specific drug delivery system based on the use of a synthetic drug glycoside prodrug composition which, when ingested by a mammal, undergoes reaction with colonic microflora to release a free drug capable of being adsorbed to or absorbed by the colonic mucosa. The present drug delivery system combines the convenience of oral administration with the specificity of topical application. It is especially useful for the treatment of inflammatory bowel disease.

The invention also provides a method of making a colon-specific prodrug comprising forming a glycosidic link between a sugar and an aglycone wherein said sugar is a sugar recognizable as a susbtrate by bacterial glycosidases produced by colonic microflora and said glycosidic link is a glycosidic link capable of being cleaved by the glycosidase enzymatic activity of colonic microflora following recognition of said sugar as a substrate by said bacterial glycosidases, and said aglycone is a drug such that the resulting glycoside is of sufficient size and hydrophilicity to allow it to pass through the mammalian gastrointestinal tract without being significantly absorbed from the gastrointestinal tract or without being significantly hydrolyzed by endogenous enzymes produced by the mammalian host so that said prodrug can reach the area of the mammalian colon where said glycosidic link can be cleaved by said bacterial glycosidases to release free drug to the area of the colon.

Further included in the invention is a prodrug formulation comprising a prodrug of the invention or a glycoside of the invention, in either case formulated for pharmaceutical or veterinary use. Such formulation will be in accordance with standard practice in the art, e.g.

the use of the appropriate unit dosage forms.

The invention further provides a method of making a glycoside which comprises forming a glycosidic link between a sugar and an aglycone wherein said sugar is a sugar recognizable as a substrate by bacterial glycosidases produced by colonic microflora and said glycosidic link is a glycosidic link capable of being cleaved by the glycosidase enzymatic activity of colonic microflora following recognition of said sugar as a substrate by said bacterial glycosidases, and said aglycone and sugar are chosen such that the resulting glycoside is not capable of being significantly hydrolyzed by endogenous enzymes produced by the mammalian host but is capable of passing substantially unabsorbed through the gastrointestinal tract to reach the colon.

Various modifications of the invention in addition to those shown and described herein will become apparent to those skilled in the art from the foregoing description and accompanying drawings. Such modifications fall within the scope of the inventive concept.

CLAIMS:

1.    A prodrug for use in a colon-specific drug delivery system comprising an aglycone attached to a sugar by means of a glycosidic link wherein said aglycone is a drug and said sugar is a sugar recognizable as a substrate by bacterial glycosidases produced by colonic microflora and said glycosidic link is a glycosidic link capable of being cleaved by the glycosidase enzymatic activity of colonic microflora following recognition of said sugar as a substrate by said bacterial glycosidases, said prodrug further being of sufficient size and hydrophilicity to allow it to pass through the mammalian gastrointestinal tract without being significantly absorbed from the gastrointestinal tract or without being significantly hydrolyzed by endogenous enzymes produced by the mammalian host so that said prodrug can reach the area of the mammalian colon where said glycosidic link can be cleaved by said bacterial glycosidases to release free drug to the area of the colon.

2.    A prodrug according to claim 1, wherein said aglycone is a steroid drug and said sugar is a simple sugar.

3.    A prodrug according to claim 2, wherein said steroid drug is prednisolone, dexamethasone, hydrocortisone or fludricortisone; said simple sugar is D-glucose, D-galactose or D-cellobiose; and said glycosidase activity of said colonic microflora is glycosidase activity produced by bacterial glycosidases which are beta-galactosidases, alpha-galactosidases, beta-galactosidases or beta-cellobiosidases.

4.    A colon-specific prodrug comprised of a drug glycoside capable of being essentially cleaved by glycosidase enzymatic activity of colonic microflora but not capable of being significantly hydrolyzed by endogenous enzymes produced by the mammalian host thus enabling the most significant amounts of free drug to be released in the area of the colon following cleavage of the drug glycoside by glycosides produced by the colonic microflora.

5. A prodrug as claimed in claim 4 which is a drug-beta-D-glycoside.

6. A glycoside which is prednisolone-21-beta-D-glucoside (PREDGLU), dexamethasone-21-beta-D-glucoside (DEXAGLU), prednisolone-21-beta-D-galactoside, dexamethasone-21-beta-D-galactoside, hydrocortisone-21-beta-D-glucoside, hydrocortisone-21-beta-D-galactoside, fludrocortisone-21-beta-D-glucoside, fludrocortisone-21-beta-D-galactoside or prednisolone-21-beta-D-cellobioside.

7. A method of making a colon-specific prodrug comprising forming a glycosidic link between a sugar and an aglycone wherein said sugar is a sugar recognizable as a substrate by bacterial glycosidases produced by colonic microflora and said glycosidic link is capable of being cleaved by the glycosidase enzymatic activity of colonic microflora following recognition of said sugar as a substrate by said bacterial glycosidases, and said aglycone is a drug such that the resulting glycoside is of sufficient size and hydrophilicity to allow it to pass through the mammalian gastrointestinal tract without being significantly absorbed from the gastrointestinal tract or without being significantly hydrolyzed by endogenous enzymes produced by the mammalian host so that said prodrug can reach the area of the mammalian colon where said glycosidic link can be cleaved by said bacterial glycosidases to release free drug to the area of the colon.

8. A method as claimed in claim 7 further defined by the specific features of claim 2 or claim 3 or wherein the resulting glycoside is one claimed in claim 6.

9. A prodrug formulation comprising a prodrug as as claimed in any one of claims 1 to 5 or a glycoside as claimed in claim 6, in either case formulated for pharmaceutical or veterinary use.

10. A method for delivering a compound to the mammalian intestine comprising administering to a mammalian hose, through said host's gastrointestinal tract, a synthetic glycoside comprising an aglycone attached to a sugar by means of a glycosidic link wherein said aglycone is the said compound and said sugar is a sugar recognizable as a substrate by bacterial glycosidases produced by mammalian intestinal microflora and said glycosidic link is a glycosidic link capable of being cleaved by the glycosidase enzymatic activity of mammalian intestinal microflora following recognition of said sugar as a substrate by said bacterial glycosidases, said glycoside further being of sufficient size and hydrophilicity to allow it to pass through the mammalian gastrointestinal tract without being significantly absorbed from the gastrointestinal tract or without being significantly hydrolyzed by endogenouse enzymes produced by the mammalian host so that said glycoside can reach the mammalian intestine where said glycosidic link can be cleaved by said bacterial glycosidases to release the free compound to the area of the intestine.

11. A method of making a glycoside which comprises forming a glycosidic link between a sugar and an aglycone wherein said sugar is a sugar recognizable as a substrate by bacterial glycosidases produced by colonic microflora and said glycosidic link is a glycosidic link capable of being cleaved by the glycosidase enzymatic activity of colonic microflora following recognition of said sugar as a substrate by said bacterial glycosidases, and said aglycone and sugar are chosen such that the resulting glycoside is not capable of being significantly hydrolyzed by endogenous enzymes produced by the mammalian host but is capable of passing substantially unabsorbed through the gastrointestinal tract to reach the colon.

1/4

FIG. I

FIG. 2

FIG. 4

FIG. 3

FIG. 5

FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | JOURNAL OF PHARMACEUTICAL SCIENCES, vol. 57, no. 12, December 1968, pages 2021-2037, Washington, D.C., US; R.R. SCHELINE: "Drug metabolism by intestinal microorganisms" * Pages 2021-2024 * | 1 | A 61 K 31/70 A 61 K 31/705 C 07 J 17/00 |
| A | FR-A-2 387 033 (UPJOHN AND THE REGENTS OF THE UNIVERSITY OF MICHIGAN) * Claims; pages 1-6 * | 1 | |
| A | EP-A-0 013 000 (DYNAPOL) * Claims * | 1 | |
| A | US-A-4 069 322 (NICOLAE S. BODOR) | | TECHNICAL FIELDS SEARCHED (Int. Cl. ³) |
| P,X | JOURNAL OF MEDICINAL CHEMISTRY, vol. 27, no. 3, March 1984, pages 261-265, American Chemical Society, Washington, D.C., US; D.R. FRIEND et al.: "A colon-specific drug-delivery system based on drug glycosides and the glycosidases of colonic bacteria" * Whole article * | 1-11 | A 61 K 31/00 C 07 J 17/00 |

The present search report has been drawn up for all claims

| Place of search | Date of completion of the search | Examiner |
|---|---|---|
| THE HAGUE | 11-07-1984 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82